# EUROPEAN PATENT SPECIFICATION

(11) **EP 3 303 630 B1**
(45) Date of publication and mention of the grant of the patent: **05.01.2022**
(21) Application number: 16730734.7
(22) Date of filing: 06.06.2016
(51) Int. Cl.: C12Q 1/6806

(54) **METHOD FOR SEPARATING DNA BY SIZE**
VERFAHREN ZUM TRENNEN VON DNS ANHAND DER GRÖSSE
PROCÉDÉ POUR SÉPARER L'ADN PAR TAILLE

(30) Priority: 05.06.2015 EP 15170885
(43) Date of publication of application: 11.04.2018
(73) Proprietor: Qiagen GmbH, 40724 Hilden (DE)
(72) Inventor: O'NEIL, Dominic, 40724 Hilden (DE); SPERLING, Tanja, 40724 Hilden (DE); GRÜNEFELD, Peter, 40724 Hilden (DE); SCHOLLE, Nicola, 40724 Hilden (DE)
(74) Representative: Hemsath, Lars
(86) International application number: PCT/EP2016/062759
(87) International publication number: WO 2016/193490

(56) References cited:
- WO-A1-99/58664
- WO-A1-2014/122288
- DE-A1- 19 943 374
- US-A- 5 898 071
- US-B1- 6 534 262
- ENGELSTEIN M ET AL: "AN EFFICIENT, AUTOMATABLE TEMPLATE PREPARATION FOR HIGH THROUGHPUT SEQUENCING", MICROBIAL AND COMPARATIVE GENOMICS, MARY ANN LIEBERT, LARCHMONT, NY, US, vol. 3, no. 4, 1 January 1998 (1998-01-01) , pages 237-241, XP000979203, ISSN: 1090-6592
- LIS J T ET AL: "Size fractionation of double stranded DNA by precipitation with polyethylene glycol", NUCLEIC ACIDS RESEARCH, INFORMATION RETRIEVAL LTD, GB, vol. 2, no. 3, 1 March 1975 (1975-03-01), pages 383-389, XP009050155, ISSN: 0305-1048 cited in the application
- K.R. PAITHANKAR ET AL: "Precipitation of DNA by polyethylene glycol and ethanol", NUCLEIC ACIDS RESEARCH, vol. 19, no. 6, 1 January 1991 (1991-01-01), pages 1346-1346, XP055203380, ISSN: 0305-1048, DOI: 10.1093/nar/19.6.1346
- V. STEIN ET AL: "An efficient method to assemble linear DNA templates for in vitro screening and selection systems", NUCLEIC ACIDS RESEARCH, vol. 37, no. 18, 17 July 2009 (2009-07-17) , pages e122-e122, XP055227621, GB ISSN: 0305-1048, DOI: 10.1093/nar/gkp589
- DEANGELIS M M ET AL: "SOLID-PHASE REVERSIBLE IMMOBILIZATION FOR THE ISOLATION OF PCR PRODUCTS", NUCLEIC ACIDS RESEARCH, INFORMATION RETRIEVAL LTD, GB, vol. 23, no. 22, 1 January 1995 (1995-01-01), XP001153688, ISSN: 0305-1048 cited in the application
- PRODELALOVA J ET AL: "Isolation of genomic DNA using magnetic cobalt ferrite and silica particles", JOURNAL OF CHROMATOGRAP, ELSEVIER, AMSTERDAM, NL, vol. 1056, no. 1-2, 12 November 2004 (2004-11-12), pages 43-48, XP004627928, ISSN: 0021-9673, DOI: 10.1016/J.CHROMA.2004.08.090

## Description

### FIELD OF INVENTION

The present invention provides a method for the size selective isolation of DNA molecules. The method of the present invention is particularly useful for to isolating target DNA molecules having a desired minimum length for preparing a sequencing library. Furthermore, a kit is provided that can be used for the size selective isolation of DNA molecules.

### BACKGROUND OF THE INVENTION

Different methods for isolating nucleic acids are well-known in the prior art. Such methods involve separating nucleic acids of interest from other sample components, such as for example protein contaminations or potentially other nucleic acids, also often referred to as non-target nucleic acids. E.g. methods for isolating nucleic acids such as DNA from various sample materials by binding them to a silica material in the presence of a chaotropic salt are well-known and established in the prior art. Exemplary methods that are based on said principle are e.g. described in EP 0 389 063, WO 03/057910, EP 0 757 106, US 6,037,465 and WO 2006/084753.

If it is intended to isolate a specific nucleic acid of interest from other nucleic acids the separation process is usually based on differences in parameters of the target and the non-target nucleic acid such as for example their topology (for example super-coiled DNA from linear DNA), their size (length) or chemical differences (e.g. DNA from RNA) and the like.

For certain applications differences in the size is an important criterion to distinguish target nucleic acids from non-target nucleic acids. E.g. size selective fractionation of DNA is an important step in the library construction for next generation sequencing (NGS) applications. Different NGS technologies and methods exist such as pyrosequencing, sequencing by synthesis or sequencing by ligation. Most NGS platforms share a common technological feature, namely the massively parallel sequencing of clonally amplified or single DNA molecules that are spatially separated in a flow cell or by generation of an oil-water emulsion. In NGS, sequencing is performed by repeated cycles of polymerase-mediated nucleotide extensions or, in one format, by iterative cycles of oligonucleotide ligation. As a massively parallel process, NGS generates hundreds of megabases to gigabases of nucleotide-sequence output in a single instrument run, depending on the platform. The inexpensive production of large volumes of sequence data is the primary advantage over conventional methods. Therefore, NGS technologies have become a major driving force in genetic research. Several NGS technology platforms have found widespread use and include, for example, the following NGS platforms: Roche/454, Illumina Solexa Genome Analyzer, the Applied Biosystems SOLiD^{™} system, Ion TorrentTM semiconductor sequence analyzer, PacBio^{®} real-time sequencing and Helicos^{™} Single Molecule Sequencing (SMS). NGS technologies, NGS platforms and common applications/fields for NGS technologies are e.g. reviewed in Voelkerding et al (Clinical Chemistry 55:4 641-658, 2009) and Metzker (Nature Reviews/ Genetics Volume 11, January 2010, pages 31-46).

Besides the feature that sequencing is performed in a massively parallel manner in NGS technologies, NGS technology platforms have in common that they require the preparation of a sequencing library which is suitable for massive parallel sequencing. Examples of such sequencing libraries include fragment libraries, mate-paired libraries or barcoded fragment libraries. Most platforms adhere to a common library preparation procedure with minor modifications before a "run" on the instrument. This procedure includes fragmenting the DNA (which may also be obtained from cDNA, e.g. by mechanical shearing, such as sonication, hydro-shearing, ultrasound, nebulization or enzymatic fragmentation followed by DNA repair and end polishing (blunt end or A overhang) and, finally, platform-specific adaptor ligation. The preparation and design of such sequencing libraries is also described e.g. in Voelkerding, 2009 and Metzker, 2010.

In order to ensure high quality sequencing data, efficient library preparation-methods are needed. Furthermore, to reduce the background in the sequencing reads, it is important to remove DNA contaminants that might be present in the sequence library as a result of the library preparation. An example of such DNA contaminants are adapter monomers and adapter-adapter ligation products that are often present in the sequencing library after adapter ligation. These contaminating small DNA molecules must be removed prior to sequencing.

To ensure efficient adaptor ligation, adaptors are used in excess during adapter ligation. Thus, after adapter ligation, unligated adaptor monomers and adaptor-adaptor ligation products such as adapter dimers are present in addition to the adaptor ligated DNA molecules. It is important to remove unligated adaptor monomers and adaptor-adaptor ligation products from the adaptor ligated DNA molecules. Otherwise, unligated adaptor monomers and adaptor-adaptor ligation products will use up sequencing capacity, thereby diminishing the power available to investigate sequences of interest. If the sequencing library comprises considerable amounts of unligated adapter monomers and adapter dimers, valuable sequencing resources are wasted. Therefore, removing unligated adaptor monomers and adaptor-adaptor ligation products increases the value of the downstream sequencing. Unligated adaptor monomers and adaptor-adaptor ligation products are usually removed by a size selective purification of the larger adaptor ligated DNA molecules, which contain the DNA fragments to be sequenced.

Several approaches were developed in the prior art in order to isolate DNA of a specific target size, respectively of a specific target size range. These size selection methods can be used in order to remove adapter dimers and monomers, as these DNA contaminations have a size that is smaller than the adapter ligated DNA molecules. A classic method for isolating DNA of a target size involves the separation of the DNA in a gel, cutting out the desired gel band(s) and then isolating the DNA of the target size from the gel fragment(s). Respective gel based size selection methods are often recommended in many next generations sequencing library preparation protocols in order to remove adapter monomers and dimers.

In addition, these methods can also be used in order to remove DNA that is larger than the desired size if contained in the sample. Thereby, it is possible to isolate DNA molecules having a size that lies within a certain size range that is determined by a lower cut-off value and an upper cut-off value. However, respective methods are time consuming, as the portion of the gel containing the nucleic acids of interest must be manually cut out and then treated to degrade the gel or otherwise extract the DNA of the target size from the gel slice.

Another widely used technology is the size selective precipitation with a poly(alkylene oxide) polymer containing buffer, in particular polyethylene glycol based buffers (Lis and Schleif Nucleic Acids Res. 1975 Mar;2(3):383-9) or the binding/precipitation on carboxyl-functionalized beads (DeAngelis et al, Nuc. Acid. Res. 1995, Vol 23(22), 4742-3; US 5,898,071 und US 5,705,628, commercialized by Beckman-Coulter (AmPure XP; SPRIselect), WO 99/58664 and US 6,534,262). The AmPure XP product is widely used and thus can be perceived as reference product. It allows to isolate DNA molecules having a size above a certain cut-off value and furthermore, allows to isolate DNA molecules having a size that lies within a specific size range (determined by the lower cut-off value and the upper cut-off value). In these methods, the carboxylated bead surface is essential in order to generate sufficient yields of the precipitated DNA fragments of the desired size. The need for surface modification of the solid phase limits the material that can be used for providing the solid support that is used for nucleic acid binding. Moreover, the surface modification is a tedious, time consuming and expensive step in the fabrication of the solid phase. Thus, even though polyethylene glycol based size selection methods are established as gold standard for size selective DNA isolation, there is a need for improved methods.

The prior art shows that there is an increasing interest and need for methods allowing the size selective isolation of DNA molecules, in particular of DNA molecules having a certain minimum size. In particular, there is a need for a simple, effective and cost-efficient method for isolating DNA of a specific minimum size and/or DNA having a size that lies within a specific size range that can be integrated into existing next generation sequencing library preparation protocols. Furthermore, there is a need for fast, simple and reliable methods for removing unligated adapter monomers and adapter dimers from adapter ligation samples, in particular adapter ligation samples obtained in the preparation of a sequencing library.

It is an object of the present invention to provide a method for isolating DNA of a target size or a target size range from a DNA containing sample comprising DNA molecules of different sizes. In particular, it is the object of the present invention to provide a poly(alkylene oxide) polymer based size selective DNA isolation method that allows to isolate target DNA molecules of a target size or target size range with sufficient yield that is independent of surface modifications of the solid support material.

### SUMMARY OF THE INVENTION

The invention can be defined by the appending claims.

The present invention is based on the established size selective DNA isolation technology, wherein at least one poly(alkylene oxide) polymer, such as a polyethylene glycol, is used in order to selectively precipitate DNA molecules having a size above a certain cut-off value from a DNA containing sample. The precipitated DNA molecules become bound to a solid support and can be recovered and removed from the remaining sample. As discussed in the background, the poly(alkylene oxide) polymer based size selective methods known in the prior art have the drawback that they require a solid phase with a modified surface in order to allow isolation of the target DNA molecules with sufficient yield. The inventors now surprisingly found that a size selective isolation of target DNA molecules with high yield is also possible when using a solid phase having an unmodified silicon containing surface, if including at least one poly(alkylene oxide) polymer which is a polyethylene glycol, at least one alkali metal salt and at least one divalent cation in the binding mixture, wherein the binding mixture comprises the divalent cation in a concentration of 3mM to 75mM, and if establishing an alkaline pH value in the binding mixture that lies in the range of 8.5 to 9.5. The examples demonstrate that the yield of the recovered DNA fragments which have a size above a certain cut-off value is significantly enhanced by this feature combination, thereby allowing to use a solid phase having an unmodified silicon containing surface for DNA binding while ensuring high recovery rates. Thereby, an effective poly(alkylene oxide) polymer based size selective DNA isolation method is provided, wherein the poly(alkylene oxide) polymer is a polyethylene glycol, that is independent of time-consuming and costly surface modifications of the solid phase.

According to a first aspect a poly(alkylene oxide) polymer based size selective DNA isolation method for isolating DNA molecules having a size above a certain cut-off value from a DNA containing sample is provided, comprising
(a) preparing a binding mixture comprising the DNA containing sample, at least one poly(alkylene oxide) polymer which is a polyethylene glycol, at least one alkali metal salt and at least one divalent cation, wherein the binding mixture comprises the divalent cation in a concentration of 3mM to 75mM, and wherein said binding mixture has a pH that lies in the range of 8.5 to 9.5 and binding precipitated DNA molecules to a solid phase having an unmodified silicon containing surface, thereby providing a solid phase having bound thereto DNA molecules having a size above the cut-off value, wherein under the used binding conditions DNA molecules having a size which is less than the cut-off value predominantly do not bind to the solid phase;
(b) separating the bound DNA molecules from the remaining sample;
   - optionally washing the bound DNA molecules; and
   - optionally eluting the bound DNA molecules from the solid phase.

In said method, DNA molecules having a size above the desired cut-off value efficiently bind to the solid phase with high yield while DNA molecules having a size below said cut-off value are predominantly not bound and thus are not recovered in step (a). The cut-off value can be adjusted by modifying the concentration of the poly(alkylene oxide) polymer which is a polyethylene glycol in the binding mixture as it is known from the prior art and also demonstrated by the examples. The presence of the divalent cation and the alkaline pH value as specified herein ensures efficient binding of the DNA molecules having a size above the cut-off value, even though a solid phase having an unmodified silicon containing surface is used. The method is particularly suitable for isolating adapter ligated DNA molecules as target DNA molecules from an adapter ligation sample and for removing adapter monomers and adapter-adapter ligation products.

According to a second aspect, a kit is provided for the size selective isolation of target DNA molecules having a size above a desired cut-off value from a DNA containing sample, wherein the kit is suitable for the method according to the first aspect, the kit comprising
(a) a binding buffer comprising at least one poly(alkylene oxide) polymer which is a polyethylene glycol, at least one alkali metal salt, at least one divalent cation and at least one buffering agent, wherein the binding buffer has a pH value that lies in the range of 8.5 to 9.5;
(b) a solid phase having an unmodified silicon containing surface;
(c) optionally at least one washing solution; and
(d) optionally at least one an elution solution.

A respective kit can be advantageously used in conjunction with and for performing the method according to the first aspect of the invention.

Other objects, features, advantages and aspects of the present application will become apparent to those skilled in the art from the following description and appended claims.

### FIGURES

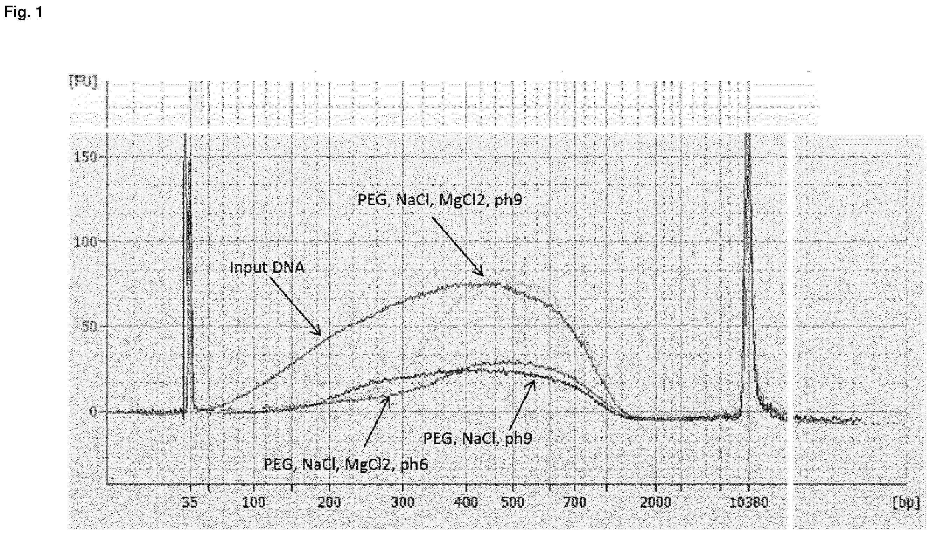

**Fig 1** shows an electropherogram (Agilent high-sensitivity DNA chip) of sheared fragmented DNA before and after size selective isolation using a binding buffer and hence binding conditions according to the invention and comparative binding buffers/conditions. The curves demonstrate that using an alkaline pH (here: 9.0) and including a divalent cation (here: from MgCl₂) in the binding mixture increases the yield of PEG 8000 precipitated DNA fragments when using a solid phase having an unmodified silica surface. FU: fluorescence units; bp: base pairs.

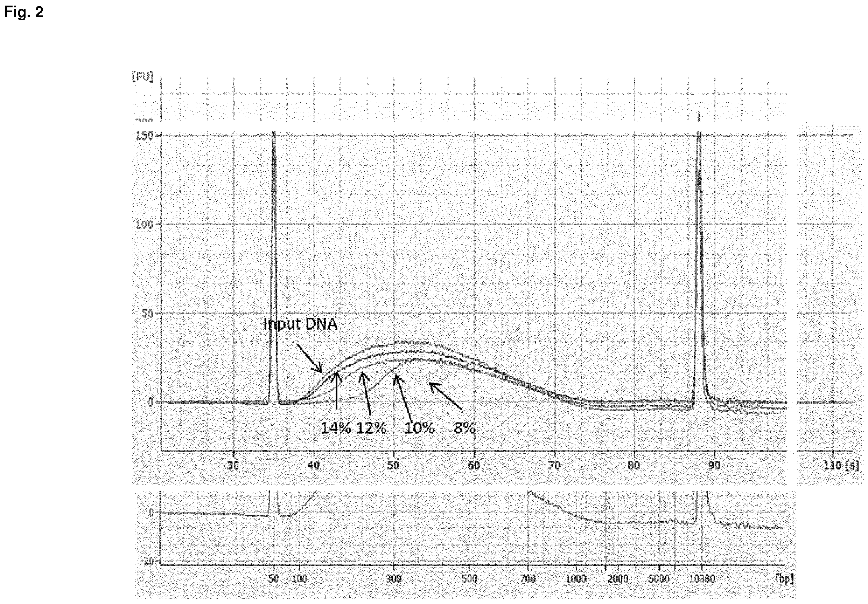

**Fig. 2** shows an electropherogram (Agilent 7500 chip) of sheared fragmented DNA before and after size selective isolation using different binding conditions according to the invention and magnetic silica particles as solid phase. As can be seen, the cut-off value of recovered DNA fragments can be varied and hence can be adjusted by using different concentrations of the poly(alkylene oxide) polymer (here: polyethylene glycol) in the binding mixture. This was achieved by adding different amounts of the binding buffer to the DNA containing sample.

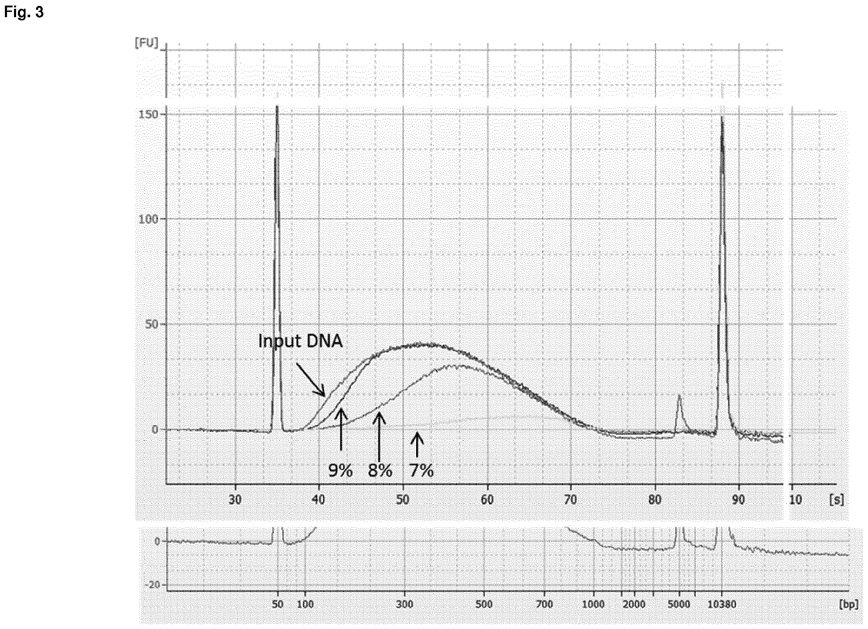

**Fig. 3** shows an electropherogram (Agilent 7500 chip) of sheared fragmented DNA before and after size selective isolation using different binding conditions according to the invention and silica spin columns as solid phase. As can be seen, the cut-off value of recovered DNA fragments can be varied and hence can be adjusted by using different concentrations of the poly(alkylene oxide) polymer (here: polyethylene glycol) in the binding mixture.

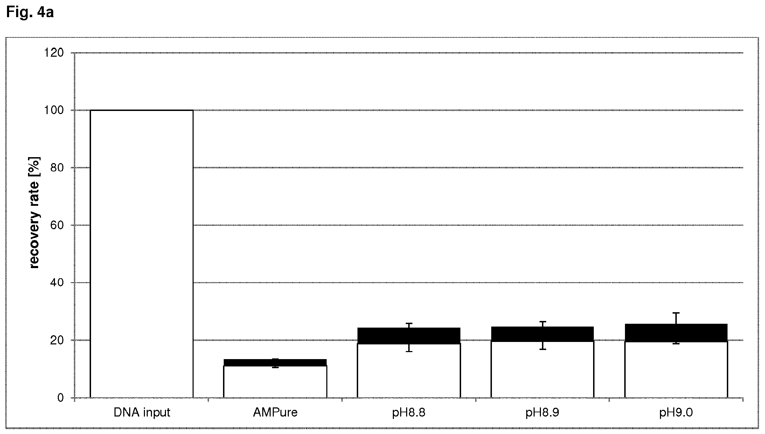

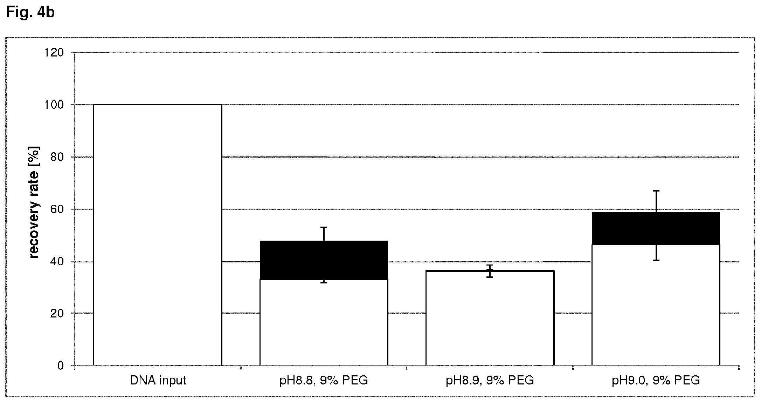

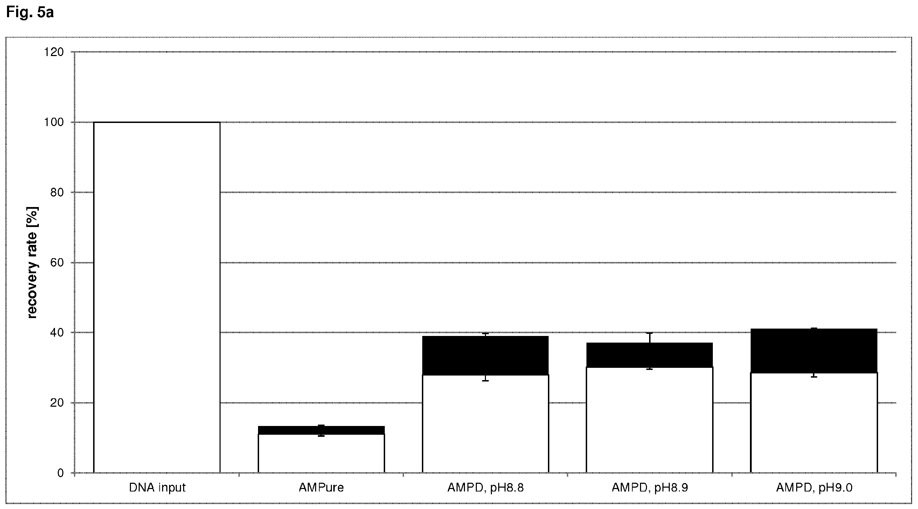

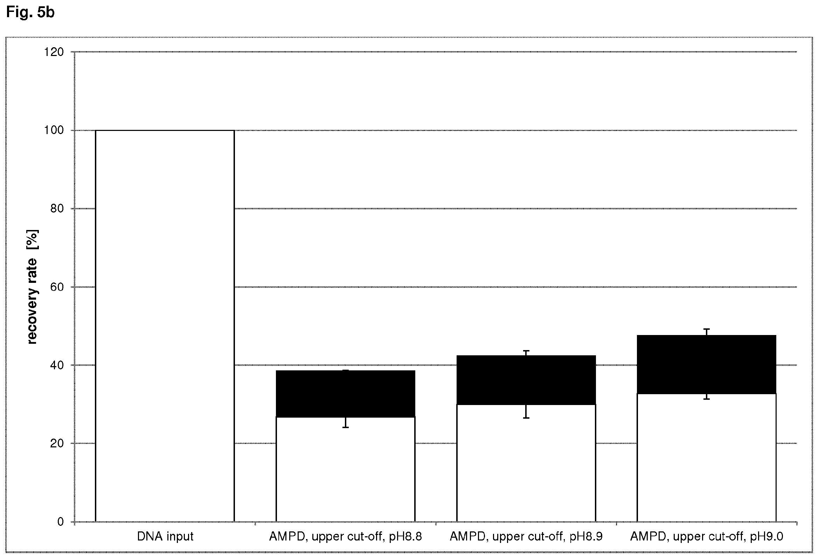

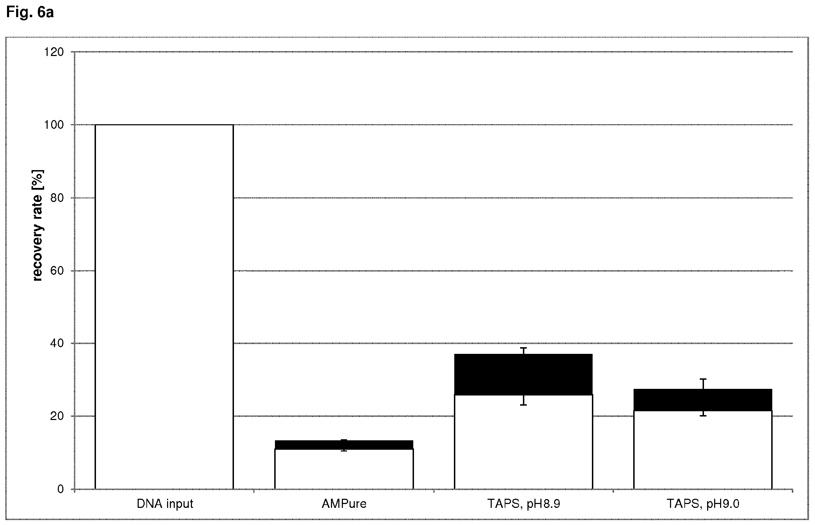

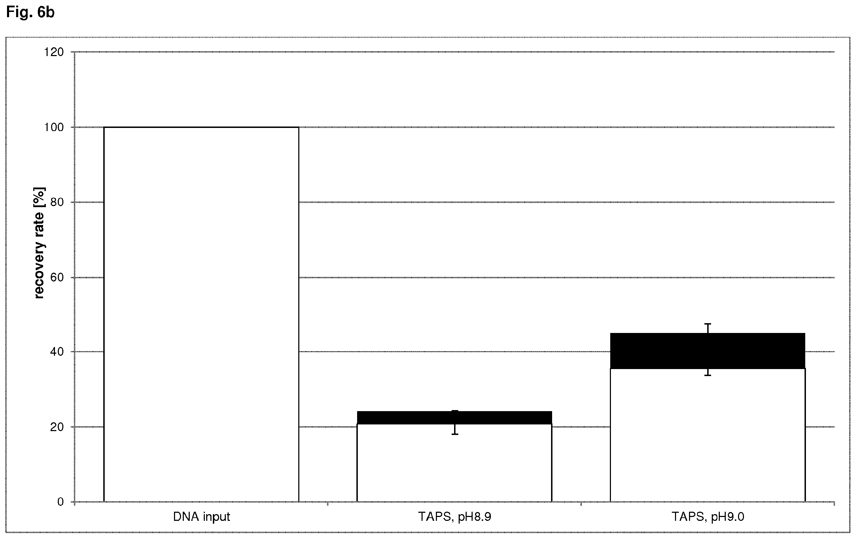

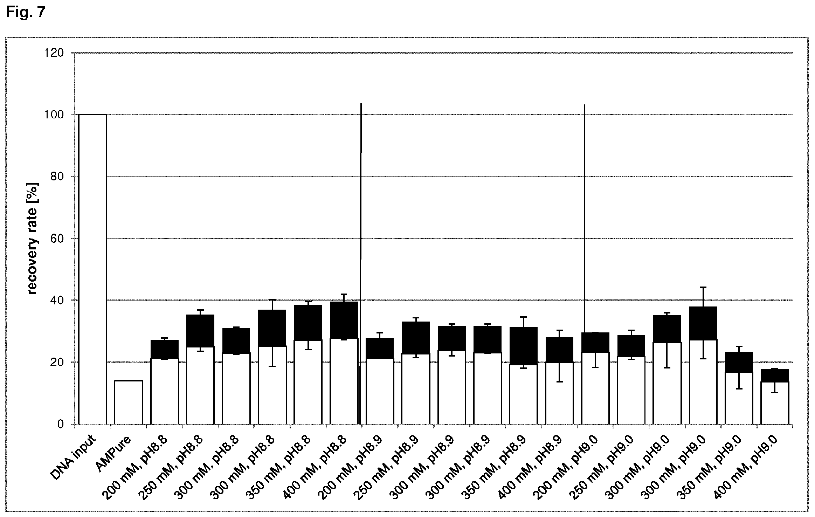

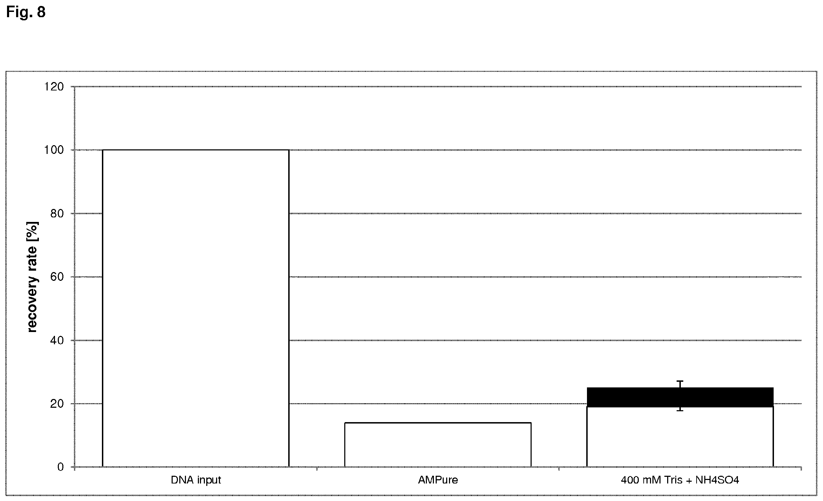

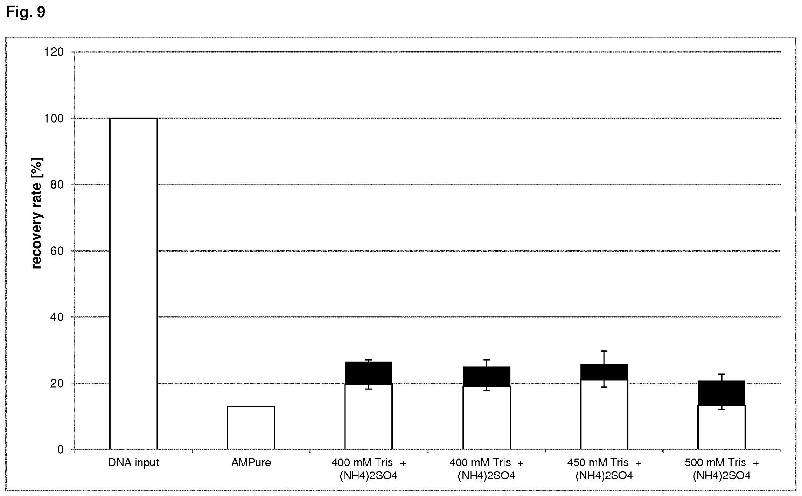

**Figs. 4 to 9** show the results of example 4, wherein different binding buffers comprising polyethylene glycol (PEG), MgCl₂, NaCI and different buffering agents at different pH values were used for performing a double-size selection to isolate DNA fragments within a range defined by an upper and lower cut-off value (bp). The results were compared to the reference product, AMPure. Black bars indicate the amount of DNA obtained after the second elution step. **Fig. 4a** shows the DNA recovery rate (%) after performing a double-size selection using CHES as buffering agent. **Fig. 4b** shows the DNA recovery rate (%) when using 9% PEG in the binding mixture at different pH values (upper cut-off value). **Fig. 5a** shows the DNA recovery rate (%) after performing a double-size selection using AMPD as buffering agent at different pH values. **Fig. 5b** shows the DNA recovery rate (%) when using 9% PEG in the binding mixture at different pH values (upper cut-off value). **Fig. 6a** and **6b** show the corresponding results when using TAPS as buffering agent. **Fig. 7** shows the DNA recovery rate (%) when performing a double-size selection using the buffering agent AMPSO in different concentrations and at different pH values. **Fig. 8** shows the DNA recovery rate (%) after performing a double-size selection using TRIS/(NH4)₂SO₄ as buffering agent. **Fig. 9** shows DNA recovery rates (%) after performing a double-size selection using different concentrations of TRIS/(NH4)₂SO₄.

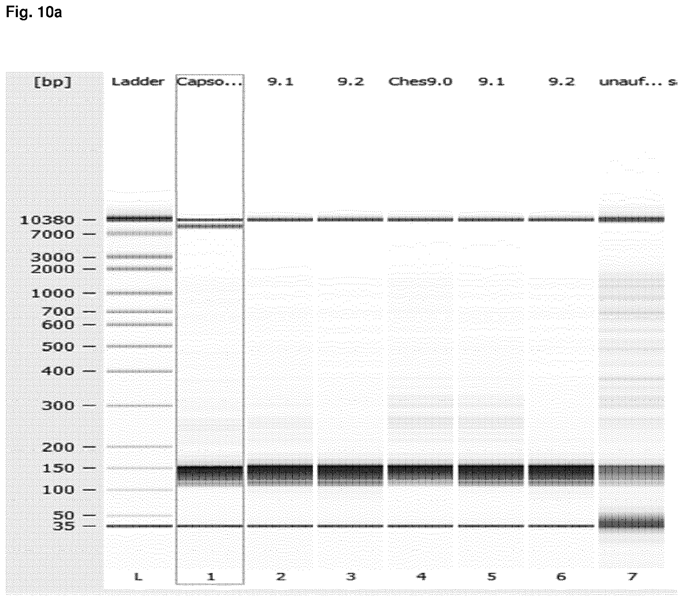

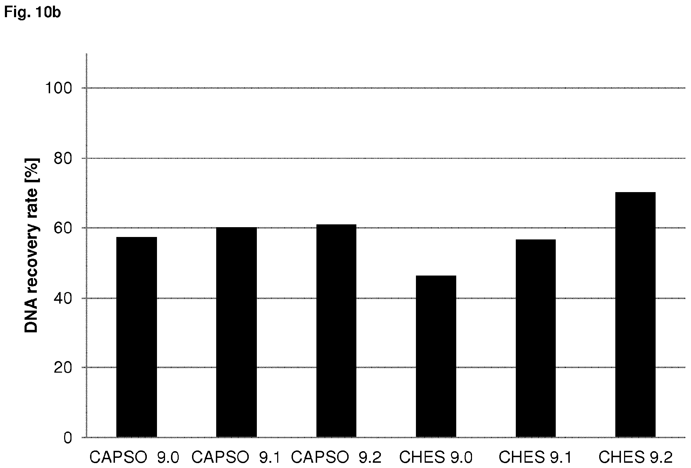

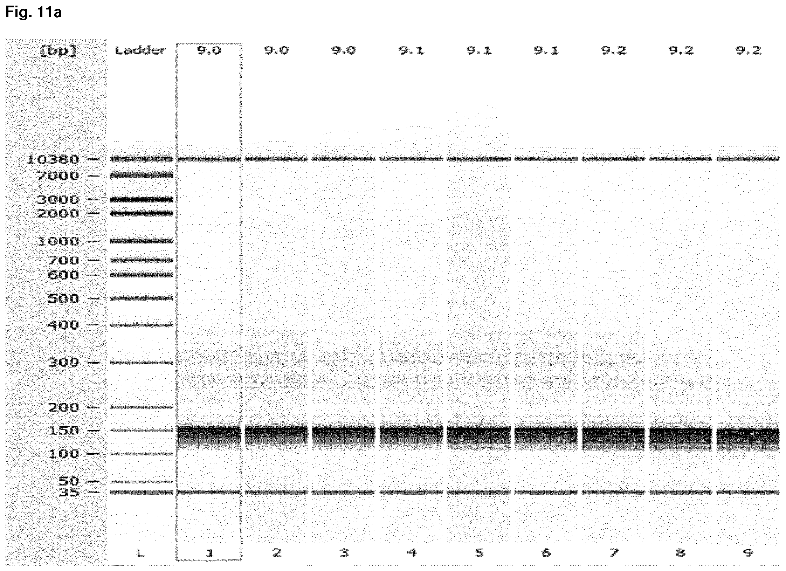

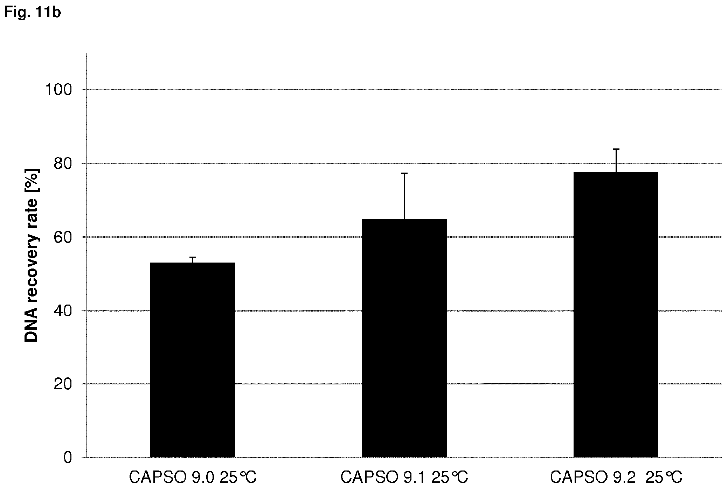

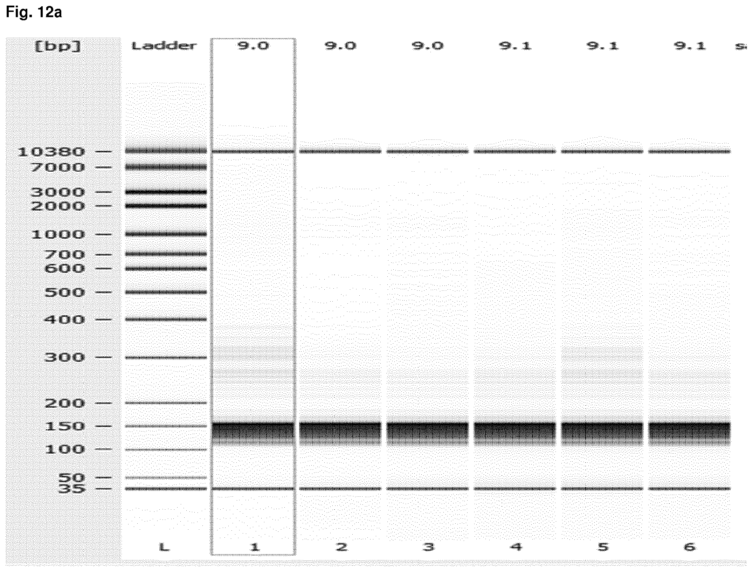

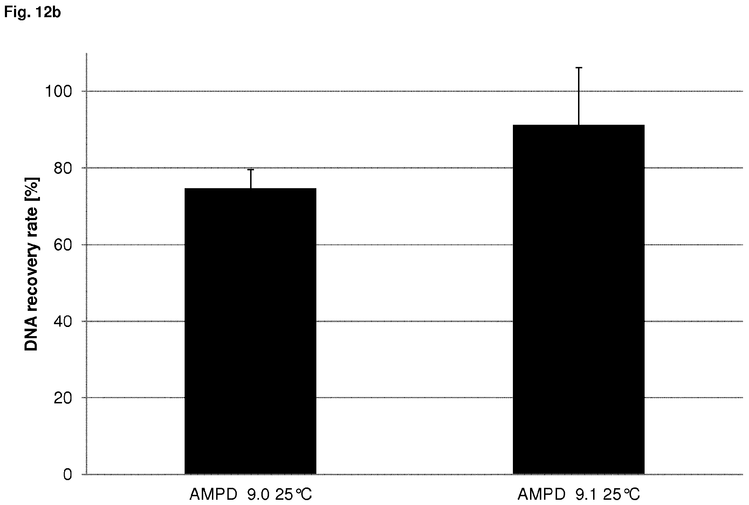

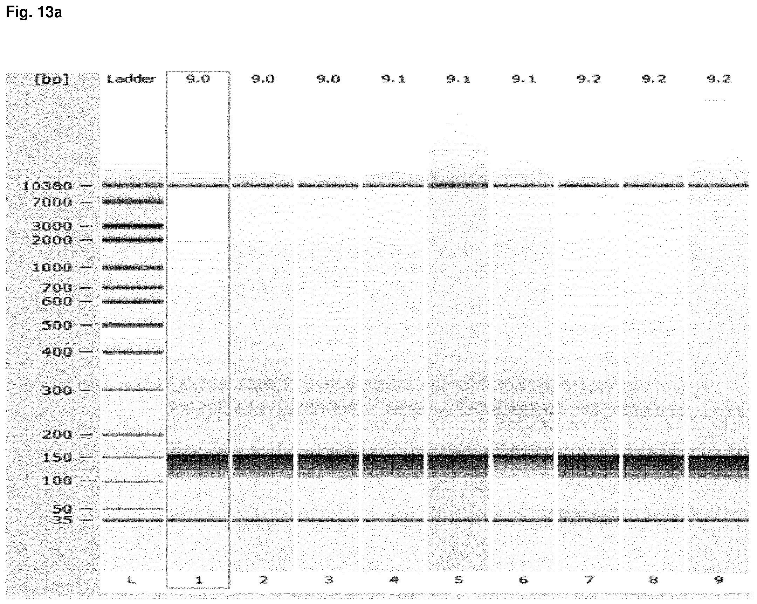

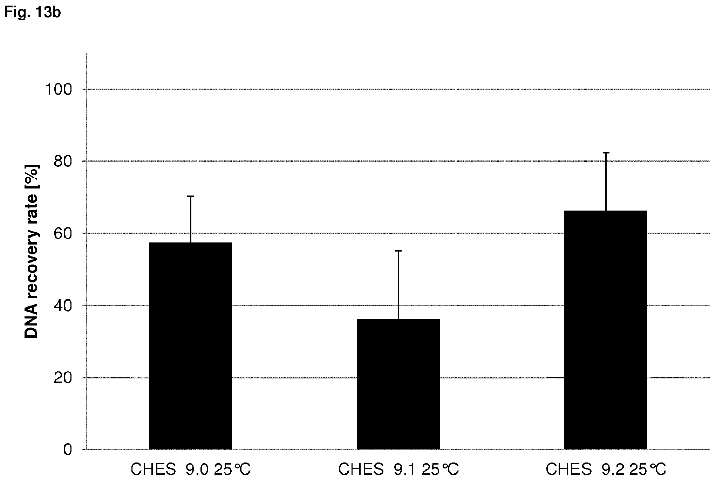

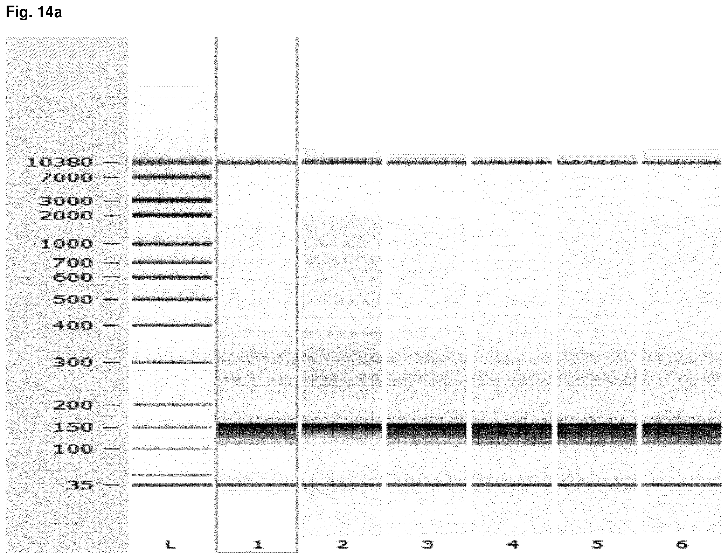

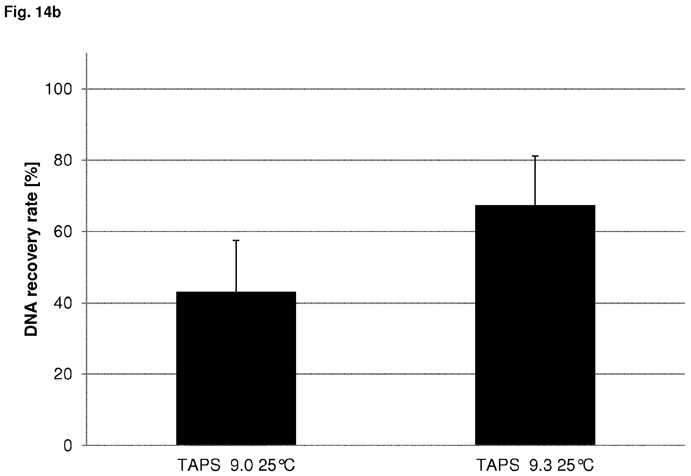

**Figs. 10 to 14** show the results of example 5, wherein different binding buffers comprising polyethylene glycol (PEG), MgCl₂, NaCI and different buffering agents at different pH values were used in cleanup and size selection in a NGS (targeted sequencing) library preparation workflow. **Fig. 10a** shows an Agilent BioAnalyzer gel-like image obtained for samples 1-6 of gene panel DNA analyzed after cleanup and size selection using freshly prepared size selection buffers comprising CAPSO or CHES as the buffering agent, adjusted to pH 9.0, 9.1 or 9.2. Sample 7 shows gene panel DNA before cleanup, 1:100 diluted. (L) indicates the ladder used. **Fig. 10b** shows the DNA recovery rate (%) calculated for samples 1-6. **Figs. 11 to 14** show results obtained for samples of gene panel DNA analyzed after cleanup and size selection using size selection buffers that have been stored for six month and that comprise different buffering agents. **Figs. 11a****,** **12a****,** **13a** **and** **14a** show the Agilent BioAnalyzer gel-like images obtained for the analyzed samples processed with the selection buffers comprising different buffering agents (**Fig. 11a**: CAPSO, pH 9.0, 9.1 or 9.2; **Fig. 12a****:** AMPD, pH 9.0 or 9.1; **Fig. 13a****:** CHES, pH 9.0, 9.1 or 9.2; **Fig. 14a****:** TAPS, pH 9.0 or 9.3). (L) indicates the ladder used. **Figs. 11b****,** **12b****,** **13b** **and** **14b** show the DNA recovery rate (%) calculated for samples processed with the respective selection buffers (**Fig. 11b**: CAPSO, pH 9.0, 9.1 or 9.2; **Fig. 12b****:** AMPD, pH 9.0 or 9.1; **Fig. 13b****:** CHES, pH 9.0, 9.1 or 9.2; **Fig. 14b****:** TAPS, pH 9.0 or 9.3).

### DETAILED DESCRIPTION OF THE INVENTION

The present invention provides a size selective method for isolating target DNA molecules by size from a DNA containing sample comprising DNA molecules of different sizes which is based on a poly(alkylene oxide) polymer which is a polyethylene glycol. In contrast to prior art methods, the method of the invention uses a solid phase having an unmodified, silicon containing surface for binding the precipitated target DNA molecules. In combination with prior art methods, the use of an unmodified solid phase was not feasible because the DNA recovery rates are very low (see examples). The present invention overcomes this problem *inter alia* because a divalent cation is present in the binding mixture and the pH of the binding mixture lies in the range of 8.5 to 9.5. This combination of features enhances the precipitation and/or binding efficiency and results in that the precipitated DNA molecules having a size above a certain cut-off value are recovered with high yield as is demonstrated by the examples. Without wishing to be bound by theory, a possible explanation for this advantageous effect on the DNA recovery is that the backbone of the DNA is more negatively charged when using an alkaline pH value during the binding/precipitation process. As a larger fraction of the surface of the solid phase having an unmodified silicon containing surface will also be more negatively charged under these conditions, the divalent ion in the binding mixture may function as a bridge between this more negatively charged surface and the negatively charged DNA thereby potentially increasing the recovery of the DNA molecules having a size above a certain cut-off value.

### METHOD

Disclosed, but not claimed, is a poly(alkylene oxide) polymer based size selective DNA isolation method for isolating DNA molecules having a size above a certain cut-off value from a DNA containing sample, comprising
(a) preparing a binding mixture comprising the DNA containing sample, at least one poly(alkylene oxide) polymer and at least one divalent cation, wherein said binding mixture has a pH that lies in the range of 8 to 10 and binding precipitated DNA molecules to a solid phase having an unmodified silicon containing surface, thereby providing a solid phase having bound thereto DNA molecules having a size above the cut-off value, wherein under the used binding conditions DNA molecules having a size which is less than the cut-off value substantially do not bind to the solid phase;
(b) separating the bound DNA molecules from the remaining sample;
   - optionally washing the bound DNA molecules; and
   - optionally eluting the bound DNA molecules from the solid phase.

As claimed, according to a first aspect a poly(alkylene oxide) polymer based size selective DNA isolation method for isolating DNA molecules having a size above a certain cut-off value from a DNA containing sample is provided, the method comprising
(a) preparing a binding mixture comprising the DNA containing sample, at least one poly(alkylene oxide) polymer which is a polyethylene glycol, at least one alkali metal salt and at least one divalent cation, wherein the binding mixture comprises the divalent cation in a concentration of 3mM to 75mM, and wherein said binding mixture has a pH that lies in the range of 8.5 to 9.5 and binding precipitated DNA molecules to a solid phase having an unmodified silicon containing surface, thereby providing a solid phase having bound thereto DNA molecules having a size above the cut-off value, wherein under the used binding conditions DNA molecules having a size which is less than the cut-off value predominantly do not bind to the solid phase;
(b) separating the bound DNA molecules from the remaining sample;
   - optionally washing the bound DNA molecules; and
   - optionally eluting the bound DNA molecules from the solid phase.

The method is in particular suitable for performing a size selection of DNA molecules during preparation of a sequencing library suitable for next generation sequencing.

The method can be performed in various embodiments. The method can be *inter alia* used to
1. Isolate desired DNA molecules having a size above a certain cut-off value (desired DNA molecules are also referred to herein as "target" DNA molecules) by binding said target DNA molecules in step (a) to the solid phase. The bound target DNA molecules are separated (step (b)) and optionally washed and eluted. In this embodiment, a "one-sided" size selection can be performed, namely for target DNA molecules having a certain minimum size as it is determined by the cut-off value. However, a maximum size of the target DNA molecules (determined by an upper cut-off value) is not defined. This embodiment is e.g. suitable for isolating and hence separating target DNA molecules having a size above a desired cut-off value from smaller DNA molecules that represent undesired contaminations such as e.g. oligonucleotides, primers, shorter DNA fragments etc.
2. Remove DNA molecules having a size above a certain cut-off value from a DNA containing sample. These longer DNA molecules are bound in step (a) to the solid phase and are in step (b) separated and hence removed from the remaining sample. The sample is thereby efficiently depleted from DNA molecules having a size above the cut-off value. The remaining sample can then be further processed, e.g. in order to isolate nucleic acids or other components that are still present in the remaining sample. The remaining sample can also be directly used in an analysis method. The DNA molecules having a size above a certain cut-off value that are bound to the solid phase can either be discarded in case they are not of interest or may be further processed, e.g. by optionally washing and eluting them.
3. Perform a size fractionation of the DNA containing sample in order to obtain one or more fractions comprising DNA molecules of a certain size range. This can be achieved by performing two or more rounds of size selection according to the teachings of the present invention. E.g. the method may comprise repeating steps (a) and (b) while increasing the concentration of the poly(alkylene oxide) polymer which is a polyethylene glycol in the binding mixture in each round. Thereby, the cut-off value is lowered in each round. E.g. in the first round, DNA molecules having a size above a certain cut-off value are bound to the solid phase and separated from the sample. In the second round, the concentration of the poly(oxyalkylene oxide) polymer which is a polyethylene glycol is increased, thereby lowering the cut-off value in the second round. DNA molecules having a size above this second cut-off value bind to the solid phase and are separated from the remaining sample. The DNA molecules bound in the second round are size selected for an upper cut-off value (determined by the first cut-off value) and a lower cut-off value (determined by the second cut-off value) and thus have a size within a defined size range. The DNA molecules that are bound to the solid phase and hence are recovered in each round and can be either discarded or eluted, e.g. after an optional washing step. This size fractionation can also be performed for three or more rounds using the same principle. This allows to size-fractionate a DNA containing sample and to provide DNA molecules of different lengths as different eluate fractions using a solid phase having an unmodified silicon containing surface, such as a silica or glass surface.

According to a preferred embodiment, the method according to the first aspect is for isolating target DNA molecules having a size within a certain size range that is determined by an upper cut-off value and a lower cut-off value from a DNA containing sample, wherein said method comprises
(a) preparing a first binding mixture comprising the DNA containing sample, at least one poly(alkylene oxide) polymer which is a polyethylene glycol, at least one alkali metal salt and at least one divalent cation, wherein the binding mixture comprises the divalent cation in a concentration of 3mM to 75mM, and wherein said first binding mixture has a pH that lies in the range of 8.5 to 9.5 and binding precipitated DNA molecules to a solid phase having an unmodified silicon containing surface, thereby providing a solid phase having bound thereto DNA molecules having a size above the upper cut-off value, wherein under the used binding conditions target DNA molecules having a size which is less than the upper cut-off value predominantly do not bind to the solid phase;
(b) separating the bound DNA molecules from the remaining sample which comprises the target DNA molecules;
(c) preparing a second binding mixture comprising the remaining sample, wherein said second binding mixture has a pH that lies in the range of 8.5 to 9.5 and wherein the concentration of the poly(alkylene oxide) polymer which is a polyethylene glycol in the second binding mixture is increased compared to the first binding mixture and binding precipitated target DNA molecules to a solid phase having an unmodified silicon containing surface, thereby providing a solid phase having bound thereto target DNA molecules having a size above the lower cut-off value, wherein under the used binding conditions DNA molecules having a size which is less than the lower cut-off value predominantly do not bind to the solid phase;
(d) separating the bound target DNA molecules from the remaining sample;
   - optionally washing the bound target DNA molecules; and
   - optionally eluting the bound target DNA molecules from the solid phase.

This embodiment is advantageous because it allows to isolate target DNA molecules having a size that lies within a defined range that is determined by the upper cut-off value and the lower cut-off value.

The individual steps (a) and (b) as well as and non-limiting suitable and preferred embodiments will now be described in detail.

In step (a), a binding mixture is prepared which comprises the DNA containing sample, at least one poly(alkylene oxide) polymer which is a polyethylene glycol, at least one alkali metal salt and at least one divalent cation, wherein the binding mixture comprises the divalent cation in a concentration of 3mM to 75mM. Importantly, the binding mixture has a pH that lies in the range of 8.5 to 9.5. Preferably, the binding mixture is prepared by contacting the DNA containing sample with a binding buffer which has a pH that lies in a range 8.5 to 9.5 and which comprises the at least one poly(alkylene oxide) polymer which is a polyethylene glycol, the at least one divalent cation and comprises at least one buffering agent. The binding buffer that is used in the present invention is also referred to herein as "precipitation buffer". The contained poly(alkylene oxide) polymer which is a polyethylene glycol precipitates DNA molecules having a size above a certain cut-off value so that they bind to the solid phase having an unmodified silicon containing surface. Thereby, a solid phase is provided having bound thereto DNA molecules having a size above a certain cut-off value. Under the used binding conditions, DNA molecules having a molecular size which is less than the cut-off value predominantly do not bind to the solid phase. As is shown by the examples and as was known in the prior art, the cut-off value can be adjusted by varying the concentration of the at least one poly(alkylene oxide) polymer which is a polyethylene glycol in the binding mixture. Thus, in step (a), DNA molecules having a size above the cut-off value efficiently adsorb to the solid phase while smaller DNA molecules having a size below the cut-off value predominantly do not bind to the solid phase having an unmodified silicon containing surface in this size selective binding step (a) and thus remain in the binding mixture.

To prepare the binding mixture, the DNA containing sample is preferably contacted with a binding buffer which has a pH that lies in the range of 8.5 to 9.5 and comprises the at least one poly(alkylene oxide) polymer which is a polyethylene glycol, the at least one divalent cation and at least one buffering agent. Preferably, the binding conditions are exclusively established by the binding buffer and no further additives or reagents are added to establish the binding conditions for binding the DNA molecules having a size above a certain cut-off value to the solid phase. This simplifies handling. Therefore, according to one embodiment, the size selective binding conditions in the binding mixture are exclusively determined by the binding buffer that is added to the DNA containing sample. However, it is within the scope of the present invention to manually adjust the pH value of the binding mixture by adding an appropriate base or acid as it is required to achieve the desired pH value in the binding mixture. The pH value of the binding mixture is important, because the pH of the binding buffer, respectively the binding mixture, significantly influences the DNA yield when using a solid phase having an unmodified silicon containing surface for binding the DNA molecules having a size above a certain cut-off value. As is demonstrated by the examples, the target DNA recovery is very low if not using an alkaline pH value during binding. In addition, the pH may also have an influence on the size of the recovered DNA molecules and hence may be used to influence the cut-off value. Therefore, it is important that the pH value of the binding mixture lies within the claimed range.

According to one embodiment, the binding mixture and/or the binding buffer that is added to the DNA containing sample to prepare the binding mixture has a pH value that lies in a range selected from 8.5 to 9.4, 8.6 to 9.3 and 8.7 to 9.2. Preferably, the pH value lies in a range of 8.6 to 9.2, more preferred 8.7 to 9.1 and most preferred 8.7 to 9.0, such as 8.8 to 8.9.

The binding mixture comprises at least one poly(alkylene oxide) polymer which is a polyethylene glycol. The polymer precipitates the DNA molecules having a size above a certain cut-off value and supports that size selective binding conditions are achieved (see also above in the background section). The term "poly(alkylene oxide) polymer" as used herein in particular refers to an oligomer or polymer of alkylene oxide units. Disclosed is that poly(alkylene oxide) polymers are known in low and high molecular weights. The molecular weight is usually a multitude of the molecular weight of its monomer(s) (e.g. 44 in case of ethylene oxide), and can range up to 50000 or even 100000. The molecular weight is indicated in Da. The poly(alkylene oxide) polymer may be linear or branched or may have other geometries. A linear poly(alkylene oxide) polymer is preferred. The poly(alkylene oxide) polymer may be unsubstituted or substituted. Substituted poly(alkylene oxide) polymers, include e.g. alkylpoly(alkylene oxide) polymers, e.g. alkylpolyethylene glycols, but also poly(alkylene oxide) esters, poly(alkylene oxide) amines, poly(alkylene oxide) thiol compounds and others. The alkylene oxide unit may be selected from the group consisting of ethylene oxide and propylene oxide. Also co-polymers such as e.g. of ethylene oxide and propylene oxide are encompassed by the term a poly(alkylene oxide) polymer. As claimed, the poly(alkylene oxide) polymer is a polyethylene glycol. Polyethylene glycol is particularly preferred because it is also commonly used in size selective DNA isolation methods as is also evidenced by the prior art discussed above. Polyethylene glycol is preferably unbranched and may be unsubstituted or substituted. Known substituted forms of polyethylene glycol include alkylpolyethylene glycols that are e.g. substituted at one or both ends with a C1-C5 alkyl group. Preferably, unsubstituted polyethylene glycol of the formula HO-(CH₂CH₂O)ₙ-H is used in the present invention. All disclosures described in this application for the poly(alkylene oxide) polymer in general specifically apply and particularly refer to the preferred embodiment polyethylene glycol, in particular unsubstituted polyethylene glycol, even if not explicitly stated.

The poly(alkylene oxide) polymer can be used in various molecular weights. According to one embodiment, the poly(alkylene oxide) polymer, which is a polyethylene glycol, has a molecular weight that lies in a range selected from 2000 to 40000, 2500 to 30000, 3000 to 25000, 3500 to 20000, 4000 to 16000, 4500 to 12000 and 5000 to 10000. Preferred is a molecular weight between 5000 to 10000, more preferred 6000 to 9000 such as 8000. Such molecular weight is particularly suitable for polyethylene glycol. Polyethylene glycol 8000 was also used in the examples.

As is demonstrated in the examples, the cut-off value is influenced and can be adjusted by the concentration of the poly(alkylene oxide) polymer which is a polyethylene glycol in the binding mixture. Therefore, by varying the concentration of the poly(alkylene oxide) polymer in the binding mixture one may relocate and thus adjust the cut-off value. Increasing the concentration of the poly(alkylene oxide) polymer in the binding mixture lowers the cut-off value. Such variation can e.g. be achieved by preparing different binding buffers each comprising the poly(alkylene oxide) polymer in a different concentration or by adding a different volume of the same binding buffer to the DNA containing sample as is also demonstrated by the examples.

The poly(alkylene oxide) polymer which is a polyethylene glycol is present in the binding mixture in a concentration sufficient to precipitate DNA molecules having a size above the cut-off value which then bind to the unmodified silica solid phase. According to one embodiment, the poly(alkylene oxide) polymer concentration in the binding mixture lies in a range selected from 6% to 20%, 7% to 17%, 7.5% to 15% and 8% to 13%. In order to ensure efficient precipitation and binding of the DNA molecules having a size above the cut-off value to the solid phase, it is preferred that the binding mixture comprises the poly(alkylene oxide) polymer in a concentration of at least 7.5%. Particularly preferred is a range of 8.5% to 12.5%. A binding buffer that is added to the DNA containing sample to prepare the binding mixture accordingly comprises the poly(alkylene oxide) polymer in an amount that achieves a corresponding concentration in the binding mixture when contacting the intended volume of the DNA containing sample with an appropriate volume of the binding buffer. The same applies in case the binding buffer is added to the remaining sample in order to establish e.g. the binding conditions for the lower cut-off value in step (c).

According to one embodiment, target DNA molecules having a size within a size range that is determined by an upper and a lower cut-off value are isolated and accordingly, at least two size selective DNA isolation steps are performed as it is described herein. According to one embodiment, the poly(alkylene oxide) polymer which is a polyethylene glycol is present in the first binding mixture in a concentration that lies in the range of 8.5% to 9.5% (for the upper cut-off value) and is present in the second binding mixture in a concentration of 10% to 12%, preferably 10.5 to 11.5% (for the lower cut-off value). As is demonstrated in the examples, this combination leads to favorable results. The binding conditions in the first and second binding mixture can be adjusted by adding different volumes of the same binding buffer.

According to one embodiment, the concentration of the at least one poly(alkylene oxide) polymer which is a polyethylene glycol in the binding buffer that is contacted with the DNA containing sample to establish the binding conditions is selected from 7% to 45%, 10% to 40%, 12.5% to 35%, 15% to 30%, 17.5% to 27.5% and 20% to 25%.

According to one embodiment, the divalent cation is provided by an earth alkaline metal, e.g. Mg²⁺.

The divalent cation may be selected from Mg²⁺, Fe²⁺, Ca²⁺, Mn²⁺, Zn²⁺ and Ni²⁺. Preferably, the divalent cation is Mg²⁺. The binding mixture and/or the binding buffer may comprise the divalent cation in form of a dissolved salt. Such salt may be e.g. a halide, sulfate, a phosphate or carbonate and preferably is a halide. Preferably, the dissolved salt is magnesium chloride.

Further salts that can be included in the binding mixture are calcium chloride and barium chloride.

The beneficial effect on DNA recovery is seen already with a low concentration of the divalent cation in the binding mixture. According to one embodiment, the divalent cation is present in the binding mixture, preferably in form of a dissolved salt, in a concentration selected from the ranges 4mM to 50mM, 5mM to 40mM, 5.5mM to 35mM, 6mM to 30mm, 6.5mM to 25mM, 7mM to 20mM and 7.5mM to 15mM. According to one embodiment, the concentration may be selected from 5mM to 20mM, 7mM to 17.5mM, 7.5mM to 15mM and 8mM to 12.5mM.

The binding mixture and/or the binding buffer comprises at least one alkali metal salt in addition to the divalent cation (which preferably is provided as dissolved salt, see above). Including a further salt can promote binding of the DNA molecules having a size above a certain cut-off value to the solid phase. This is particularly feasible if the divalent cation is used in a lower concentration. The alkali metal salt preferably is a halide. It is preferably selected from sodium chloride, potassium chloride, lithium chloride and cesium chloride. More preferably, the salt is sodium chloride. According to one embodiment a non-chaotropic salt is used as additional salt. According to one embodiment, the binding mixture does not comprise a chaotropic salt such as guanidinium salts, iodides, thiocyanates, perchlorates or other chaotropic salts of equal or stronger chaotropic nature.

The binding mixture may comprise the additional salt in a concentration selected from ≥ 250mM, ≥ 350mM, ≥ 500mM, ≥ 600mM, ≥ 650mM, ≥ 700mM, ≥ 750mM and ≥ 800mM. The concentration can be selected e.g. from 250mM to 3M, 250mM to 2M, 350mM to 1.75M, 500mM to 1.5M, 600mM to 1.3M and 650mM to 1.25M. The binding buffer may comprise the salt in a concentration selected from 0.5M to 4M, 0.7M to 3.5M, 1M to 3M, 1.2M to 2.75M and 1.3M to 2.5M. The salt is preferably a halide such as NaCI or KCI.

According to one embodiment, the binding mixture and/or the binding buffer comprises the divalent cation in form of a dissolved salt, preferably MgCl₂, and additionally comprises an alkali metal salt, preferably a halide, more preferably sodium chloride. As is demonstrated by the examples, this combination leads to particularly good results.

According to one embodiment, after contacting the binding buffer with the DNA containing sample, a pH value is provided in the resulting binding mixture that corresponds to or substantially corresponds to the pH value of the binding buffer. Thus, according to one embodiment, the pH value of the binding buffer is substantially maintained in the resulting binding mixture. This is achieved, respectively is supported, by the at least one buffering agent that is comprised in the binding buffer. Preferably, the pH value in the binding mixture does not deviate by more than +/- 0.2 pH units, preferably not more than +/- 0.15 pH units, more preferred not more than +/- 0.1 pH units, most preferred not more than +/- 0.05 pH units from the pH value of the binding buffer.

The binding conditions (and hence the cut-off value) can be and preferably are controlled by the binding buffer that is added to the DNA containing sample. According to one embodiment, the binding conditions such as the concentration of the poly(alkylene oxide) polymer which is a polyethylene glycol, the divalent cation and the binding pH value are established by contacting the DNA containing sample with the binding buffer. Preferably, no further adjustments are made to establish the binding conditions in the binding mixture. Thus, preferably, the binding mixture is provided exclusively by contacting the binding buffer with the DNA containing sample but no further buffers or other reagents are added to establish the binding conditions for binding the precipitated target DNA molecules to the solid phase having an unmodified surface. This advantageously avoids handling and adjustment errors. E.g. 1 volume of DNA containing sample can be contacted with 0.1 to 10 volumes of binding buffer, 0.15 to 5 volumes of binding buffer, preferably 0.15 to 3 volumes of binding buffer, more preferred 0.2 to 2 volumes of binding buffer, most preferred 0.2 to 1 volume of binding buffer. Contacting the binding buffer with the DNA containing sample reduces the concentration of the ingredients contained in the binding buffer in the resulting binding mixture due to a dilution effect. As is demonstrated in the examples, by adding e.g. different amounts of the binding buffer to the DNA containing sample, one may flexibly adjust and thus relocate the cut-off value.

Even though it is preferred for many applications that the pH in the binding mixture is established by contacting the DNA containing sample with the binding buffer, the present invention also covers embodiments, wherein the pH value in the binding mixture is adjusted and thus modified after the DNA containing sample was contacted with the binding buffer. Thus, according to one embodiment, the pH value of the binding mixture is adjusted to the binding pH value which lies in the range of 8.5 to 9.5. Suitable pH values are described above and it is referred to the respective disclosure. E.g. the adjustment can be made manually. The pH value of the binding mixture may be determined and then adjusted to the desired alkaline binding pH value by adding appropriate pH modifying substances such as acids or bases. Such procedure can be advantageous if the DNA containing sample has an unusual high or low pH value. However, preferably, the pH of the binding mixture is exclusively established by the addition of the binding buffer.

As is described above, the binding mixture is preferably prepared by contacting the DNA containing sample with a binding buffer. The binding buffer comprises a buffering agent. The buffering agent supports that the pH value of the binding mixture is maintained at the desired alkaline binding pH value which is as described important in order to ensure in combination with the divalent cation good recovery rates of the target DNA molecules when using a solid phase having an unmodified silicon containing surface. Therefore, it is important that the pH value of the binding buffer, which also determines the pH value of the binding mixture, is sufficiently stable to reliably provide the desired binding pH. The buffering agent is chosen such that it has a buffering capacity that includes the desired binding pH value. As a rough estimate, a useful buffering capacity of a buffering agent is often pKa ± 1. Suitable buffering agents are well-known in the prior art and include but are not limited to TRIS, MOPS, BICINE, TRICINE, CHES, CAPSO, TAPS, AMPSO, AMPD and (NH4)₂SO₄. Suitable buffering agents include but are not limited to TRIS, MOPS, BICINE, TRICINE, CHES, TAPS, AMPSO, AMPD and (NH4)₂SO₄. Also combinations of buffering agents may be used as is demonstrated by the examples. According to one embodiment, AMPSO and/or a combination of Tris/(NH₄)₂SO₄ is used a buffering agent.

According to one embodiment, the binding buffer comprises the at least one buffering agent in a concentration selected from 25mM to 1M, 50mM to 750mM, 75mM to 700mM, 100mM to 650mM, 125mM to 600mM, 150mM to 550mM, 175mM to 525mM and 200mM to 500mM. It is preferred to use higher concentrations of the at least one buffering agent in the binding buffer, preferably at least 50mM, at least 75mM, at least 100mM, at least 150mM, at least 175mM, or at least 200mM. This ensures a reliable buffering capacity even if a larger amount of DNA containing sample is processed and/or if two or more rounds of size selection are performed. This reduces or avoids changes in the pH value of the binding mixture that is created when contacting the sample with the binding buffer. Accordingly, also the binding mixture comprises a buffering agent. The binding mixture may comprise the buffering agent or combination of buffering agents in a concentration selected from 25mM to 600mM, 50mM to 500mM, 60mM to 450, 70mM to 400mM, 80mM to 350mM, 90mM to 300mM and 100mM to 275mM. If more than one buffering agent is used, the indicated concentrations/ranges refer to the total concentration of buffering agents. The optimal concentration may also depend on which buffering agent or combination of buffering agent is used and can be determined by the skilled person following the principles described herein.

According to one embodiment, step (a) comprises: preparing a binding mixture comprising the DNA containing sample, at least one poly(alkylene oxide) polymer, which is polyethylene glycol, at least one alkali metal salt and at least one divalent cation, wherein the binding mixture comprises the divalent cation in a concentration of 3mM to 75mM, and wherein said binding mixture has a pH that lies in the range of 8.5 to 9.5 by contacting the DNA containing sample with a binding buffer comprising the at least one poly(alkylene oxide) polymer which is a polyethylene glycol, the at least one divalent cation and at least one buffering agent, wherein the binding buffer has a pH that lies in a range of 8.5 to 9.5 and binding precipitated DNA molecules to a solid phase having an unmodified silicon containing surface, thereby producing a solid phase having bound thereto DNA molecules having a size above the cut-off value, wherein under the used binding conditions DNA molecules having a molecular size which is less than the cut-off value predominantly do not bind to the solid phase. As is described above, the poly(alkylene oxide) polymer is polyethylene glycol, more preferred unsubstituted polyethylene glycol, and the divalent cation is preferably Mg²⁺. It may be comprised in the binding mixture in a concentration that lies in the range of 5mM to 50mM, preferably 7mM to 25mM. Moreover, the binding mixture preferably comprises an additional salt such as sodium chloride or potassium chloride, sodium chloride being preferred. Suitable concentration ranges were described above. The pH value of the binding mixture is in the range of 8.5 to 9.5, particularly preferred pH values are also described above.

According to one embodiment the binding buffer that is added to the DNA containing sample to prepare the binding mixture has the following characteristics:
i) it comprises Mg²⁺ as divalent cation, preferably in form of MgCl₂;
ii) it comprises polyethylene glycol in a concentration selected from 7% to 45%, 10% to 40%, 12.5% to 35%, 15% to 30%, 17.5% to 27.5% and 20% to 25%;
iii) it comprises an alkali metal salt, preferably sodium chloride, in a concentration that lies in the range of 0.5M to 2.75M, preferably 0.75M to 2.5M;
iv) it comprises the at least one buffering agent in a concentration that lies in a range of 100 to 550mM;
v) it has a pH that lies in the range of 8.6 to 9.2.

The average length of the DNA molecules that under the chosen binding conditions bind to the solid phase having an unmodified silicon containing surface lies above the cut-off value while the average length of the DNA molecules which are not bound to the solid phase lies below the cut-off value. The expression that "DNA molecules having a size above the cut-off value bind to the solid phase" and similar expressions used herein, in particular specify that DNA molecules having a size at the cut-off value or above bind to the solid phase. I.e. if the cut-off value is described as being 100nt, this means that DNA molecules having a size of 100nt or larger bind to the solid phase. Thus, the cut-off value in particular defines the size of the smaller DNA molecules that substantially do not bind under the respective binding conditions to the solid phase. According to one embodiment, the cut-off value refers to the length of the shortest DNA fragment that can be visualized by electropherogram. According to one embodiment, the cut-off value corresponds to the point where the curve of the electropherogram meets the x-axis. It is pointed out though that at this point, respectively this cut-off value, there usually is no quantitative recovery of the DNA but the percentage of captured DNA molecules increases with increasing size of the DNA molecules.

The DNA containing sample comprises DNA molecules of different sizes (lengths). The DNA containing sample may comprise single-stranded and/or double stranded DNA. The method according to the present invention allows size selection of single stranded as well as of double-stranded DNA. Preferably, the DNA molecules are linear, double-stranded DNA molecules. The DNA containing sample can be of various origins, including but not limited to biological samples and artificial samples that were obtained during nucleic acid processing. According to one embodiment, the DNA containing sample is not a lysate. According to one embodiment, the DNA containing sample is a sample of extracted DNA or extracted DNA that has been further processed, e.g. by shearing or by way of an enzymatic reaction. According to one embodiment, the DNA containing sample was obtained after an enzymatic reaction. Exemplary enzymatic reactions that provide DNA containing samples that can be processed using the size selective DNA isolation method of the invention include but are not limited to amplification reactions, ligase reactions, in particular adapter ligation reactions and polynucleotide, e.g. poly A, tailing reactions. According to one embodiment, the DNA containing sample comprises fragmented DNA, such as e.g. sheared DNA. According to one embodiment, the DNA containing sample comprises sheared genomic DNA or sheared cDNA. According to one embodiment the DNA containing sample is a solution resulting from a size shearing procedure. Such DNA containing sample comprises DNA fragments of different sizes. Said fragmented DNA may also be end-repaired to provide DNA fragments having blunt ends. Thus, according to one embodiment, the DNA containing sample comprises linear, blunt-ended DNA fragments of different sizes. According to a preferred embodiment, the DNA containing sample was obtained during the preparation of a sequencing library, in particular during preparation of a next generation sequencing library. According to one embodiment, the DNA containing sample is an adapter ligation sample that was obtained as a result of an adapter ligation step, e.g. during preparation of a sequencing library. According to a preferred embodiment, the DNA containing sample is an adapter ligation sample which comprises (i) double-stranded DNA molecules that are flanked 5' and/or 3' by adapters, (ii) adapter monomers and (iii) adapter-adapter ligation products such as e.g. adapter dimers. Furthermore, the DNA containing sample may comprise additional contaminating components such as e.g. mono, oligo-and/or polynucleotides and proteins such as enzymes that are e.g. still present in the DNA containing sample from previous enzymatic sequencing library processing steps. The method according to the present invention allows *inter alia* to size selectively purify double-stranded DNA molecules that are flanked 5' and/or 3' by adapters, preferably are flanked at their 5' end and their 3' end by adapters, thereby efficiently removing respective contaminants. According to one embodiment, the DNA containing sample comprises amplification products, e.g. PCR products. Thus, according to one embodiment, the DNA containing sample is a sample resulting from an amplification procedure, in particular resulting from a PCR amplification.

According to one embodiment, the DNA containing sample has one or more of the following characteristics:
i) the DNA containing sample is a sample of extracted DNA or extracted DNA that has been further processed, e.g. by shearing or by way of an enzymatic reaction;
ii) the DNA containing sample was obtained after an enzymatic reaction, preferably an amplification reaction or ligase reaction, in particular after an adapter ligation reaction;
iii) the DNA containing sample comprises fragmented DNA, e.g. sheared DNA;
iv) the DNA containing sample comprises linear, blunt-ended DNA fragments of different sizes;
v) the DNA containing sample comprises amplification products, preferably PCR products;
vi) the DNA containing sample is an adapter ligation sample that was obtained as a result of an adapter ligation step; and/or
vii) the DNA containing sample is an adapter ligation sample which comprises (i) double-stranded DNA molecules that are flanked 5' and/or 3' by adapters, (ii) adapter monomers and (iii) adapter-adapter ligation products such as e.g. adapter dimers.

The binding conditions and in particular the concentration of the poly(alkylene oxide) polymer which is a polyethylene glycol in the binding mixture is chosen such that a desired cut-off value is obtained that allows e.g. to remove undesired small DNA molecules such as e.g. adaptors, adaptor-adaptor ligation products and primers. In the method according to the invention, DNA molecules having a size above the cut-off value are efficiently bound to the solid phase and hence can be recovered or removed during size selection. The precise cut-off value to be chosen depends e.g. on the intended use of the target DNA molecules and also the size of e.g. contaminating small DNA molecules that are not supposed to be bound and thus recovered during size selection. The removal of small DNA molecules that is achieved with the present invention does not necessarily have to be complete. For several applications it is sufficient that unwanted small DNA molecules are depleted to an extent that they do not significantly disturb or hamper the intended downstream reaction. As described, however, it is often not necessary that 100% of the smaller DNA is removed. As described, the method can also be performed in order to size selectively isolate such DNA fragments having a length within a certain size range that is determined by an upper and lower cut-off value by performing at least two size selective binding steps.

The sizes, respectively cut-off values indicated herein with reference to nucleotides "nt", refer to the chain length of the DNA molecules and thus are used in order to describe the length of, respectively describe the cut-off value for single-stranded as well as double-stranded DNA molecules. In double-stranded DNA molecules said nucleotides are paired. Hence, if the DNA is a double stranded molecule, what is preferred, the above indications with respect to the size or length in "nt" refers to "bp". Thus, if a double-stranded DNA molecule has a chain length, respectively size of 100nt, said double-stranded DNA molecule has a size of 100bp. The same applies to the definition of the cut-off value for double-stranded DNA molecules.

A (lower) cut-off value that lies in the range of 125nt to 200nt, preferably 135nt to 200nt such as e.g. 150nt to 190nt is e.g. particularly suitable for size selection during the preparation of a sequencing library. Here, a size selective DNA isolation step is in particular performed in order to separate adapter ligated DNA molecules from unligated adapter monomers and adapter-adapter ligation products, such as adapter dimers. The size of adapters that are commonly used for preparing sequencing libraries for next generation sequencing often lies in the range of 25nt to 75nt, in particular 30nt to 60nt. For removing unligated adapter monomers and adapter-adapter ligation products (such as in particular adapter dimers), the cut-off value is according to one embodiment chosen such that it lies above the size of the adapter monomer(s) and above the size of the expected adapter-adapter ligation product(s). Preferably, the cut-off value is at least 10nt, preferably at least 15nt, at least 20nt, at least 25nt or at least 30nt larger than the expected size of adapter-adapter ligation product(s) in order to ensure an efficient removal of the adapter monomer(s) and adapter-adapter ligation product(s). According to one embodiment, such a cut-off value in the range of 125nt to 200nt and the preferred subranges described above represents the lower cut-off value in a method wherein target DNA molecules having a size within a certain size range that is defined by an upper and lower cut-off value are isolated. According to this embodiment, the upper cut-off value may lie in a range of 250nt to 1000nt, 275nt to 750nt and 300nt to 500nt. Advantageously, the upper and the lower cut-off value can be easily adjusted by adding appropriate amounts of the binding buffer to establish size selective binding conditions in the binding mixture.

In the binding steps (step (a) and optional further binding step(s)), DNA molecules having a size above the desired cut-off value bind to the solid phase having an unmodified silicon containing surface, which preferably provides a siliceous surface for DNA binding. According to one embodiment, at least 75%, at least 80%, preferably at least 85%, more preferred at least 90%, more preferred at least 95% or more preferred at least 97% of the DNA molecules having a size above the cut-off value bind in step (a) (and step (c) if performed). According to one embodiment, not more than 25%, not more than 20%, not more than 15%, not more than 10%, not more than 7% or not more than 5% of the DNA molecules having a size below the cut-off value bind to the solid phase in step (a) (and further binding step(s) if performed).

The solid phase comprises an unmodified silicon containing surface to which the precipitated target DNA molecules having a size above the cut-off value bind under the chosen binding conditions. Binding is in particular achieved by adsorption. Preferably, the solid phase provides a siliceous surface such as a silica surface. The term "silica surface" as used herein includes surfaces comprising or consisting of silicon dioxide and/or other silicon oxides, diatomaceous earth, glass, silica gel, zeolithe, bentonite, alkylsilica, aluminum silicate and borosilicate. The solid phase has an unmodified silicon containing surface. Therefore, the surface is not modified with nucleic acid binding ligands or other nucleic acid binding groups. E.g., the solid phase does not carry ligands at its binding surface that comprise ion exchange groups, in particular, the surface of the solid phase is not modified with functional ligands. In particular, it is not modified with ligands comprising anionic or cationic exchange groups such as e.g. amine groups or carboxyl groups as it is common in prior art methods to ensure a good yield. According to one embodiment, the silica surface does not comprise any functional groups besides its silanol groups or other oxidized forms of silicon, like oxides. Exemplary solid phases that can be used in conjunction with the present invention include, but are not limited to, solid phases comprising an unmodified silica surface, including but not limited to, silica particles, silica fibres, glass materials such as e.g. glass powder, glass fibres, glass particles or controlled pore glass, silicon dioxide, glass or silica in particulate form such as powder, beads or frits. The term solid phase is not intended to imply any limitation regarding its form or design. Thus, the term solid phase encompasses appropriate materials having an unmodified silicon containing surface, in particular an unmodified silica surface that is porous or non-porous; permeable or impermeable. Suitable solid phases include but are not limited to membranes, filters, sheets, particles, magnetic particles, beads, gels, powders, fibres and the like. Particularly preferred is the use of silica such as silica gel, and polysilicic acid materials, borosilicates, silicates and anorganic glasses as solid phase. The solid phase comprising an unmodified silica surface may e.g. have the form of a filter, fibres, membrane or particles. According to the present invention, the use of a column based solid phase or the use of particles, in particular magnetic particles, is preferred.

According to one embodiment, silica particles having an unmodified silica surface are used that may have the form of beads. Preferably, said particles have a size of about 0.02 to 30 µm, 0.1 to 15 µm, 0.2 to 12.5µm, 0.5 to 10 µm and 0.75 to 7µm. To ease the processing of the nucleic acid binding solid phase, preferably magnetic silica particles are used. Magnetic particles respond to a magnetic field. The magnetic silica particles may e.g. be ferrimagnetic, ferromagnetic, paramagnetic or superparamagnetic. Suitable magnetic silica particles are for example described in WO 01/71732, WO 2004/003231 and WO 2003/004150. Other magnetic silica particles are also known from the prior art and are e.g. described in WO 98/31840, WO 98/31461, EP 1 260 595, WO 96/41811 and EP 0 343 934 and also include for example magnetic silica glass particles. The use of magnetic particles has advantages, because the magnetic particles including the bound DNA can be processed easily by the aid of a magnetic field, e.g. by using a permanent magnet. This embodiment is e.g. compatible with established robotic systems capable of processing magnetic particles. Here, different robotic systems exist in the prior art that can be used in conjunction with the present invention to process the magnetic silica particles to which the target DNA molecules were bound. According to one embodiment, magnetic particles are collected at the bottom or the side of a reaction vessel and the remaining liquid sample is removed from the reaction vessel, leaving behind the collected magnetic particles to which the DNA molecules are bound. Removal of the remaining sample can occur by decantation or aspiration. Such systems are well known in the prior art and thus need no detailed description here. In an alternative system that is known for processing magnetic particles the magnet which is usually covered by a cover or envelope plunges into the reaction vessel to collect the magnetic particles. As respective systems are well-known in the prior art and are also commercially available (e.g. QIASYMPHONY^{®}; QIAGEN), they do not need any detailed description here. In a further alternative system that is known for processing magnetic particles, the sample comprising the magnetic silica particles can be aspirated into a pipette tip and the magnetic particles can be collected in the pipette tip by applying a magnet e.g. to the side of the pipette tip. The remaining sample can then be released from the pipette tip while the collected magnet silica particles which carry the bound target DNA molecules remain due to the magnet in the pipette tip. The collected magnetic particles can then be processed further. Such systems are also well-known in the prior art and are also commercially available (e.g. BioRobot EZ1, QIAGEN) and thus, do not need any detailed description here.

According to one embodiment, the solid phase is comprised in a column. The term "column" as used herein in particular describes a container having at least two openings. Thereby, a solution and/or sample can pass through said column. The term "column" in particular does not imply any restrictions with respect to the shape of the container which can be e.g. round or angular and preferably is cylindrical. However, also other shapes can be used, in particular when using multi-columns. The column comprises the solid phase having an unmodified silicon containing surface that is used for DNA binding. Said solid phase comprised in the column should allow the passage of a solution, respectively the binding mixture when applied to the column. This means that if e.g. a centrifuge force is applied to the column, a solution and/or the binding mixture is enabled to pass through the column in direction of the centrifuge force. As discussed above, when using a respective column based DNA isolation procedure, the binding mixture is usually passed through the column, e.g. assisted by centrifugation or vacuum, and the DNA molecules having a size above the cut-off value bind to the comprised solid phase during said passage. The column can be used in a single format or in a multi-format. Such multi-columns having a similar format as multi-well plates and which comprise a solid phase such as a silica membrane or glass fibres, are well-known in the prior art and are also commercially available. Preferably, the column is a spin column. Preferably, a DNA binding membrane or DNA binding fibres providing a silicon containing surface are used as solid phase. Examples include but are not limited to silica membranes, glass fibre membranes or filters providing a silicon containing surface for DNA binding. Preferably, the membrane is porous. For a column based procedure, it is preferred to use a membrane or fibres as solid phase which comprise or consist of silica in the column. As is shown in the examples, respective column materials are also suitable for precisely adjusting the cut-off value within a narrow range. Suitable and preferred silica based materials which provide a silica surface suitable for DNA binding were also described above. A further common solid phase comprised in a column is a fill of silica particles, or a layer of a silica material (e.g. a silica gel). E.g. the silica particles can be arranged as a layer on an inert filter or membrane, thereby forming a DNA solid phase. To alleviate the passage of the binding mixture through the solid phase comprised in the column, suitable means can be used such as e.g. centrifugation or the use of a pressure difference-generating apparatus which e.g. presses the sample through the column, respectively the solid phase or sucks it through the solid phase by applying a vacuum. Respective means are well known in the prior art and thus need no further description here.

Contacting the DNA containing sample with the binding buffer to provide the binding mixture and binding of the DNA molecules having a size above a cut-off value to the solid phase may be performed simultaneously or sequentially. According to one embodiment, the DNA containing sample is contacted with the binding buffer and the resulting binding mixture is then contacted with the solid phase. This embodiment is e.g. feasible if a column-based isolation protocol is used. A likewise approach can also be used when using a particulate solid phase such as e.g. magnetic silica particles. When using a particulate solid phase, the solid phase, the binding buffer and the DNA containing sample can be added in any order. E.g. it is within the scope of the present invention to first provide the solid phase and the binding buffer and then add the sample or to first provide the sample, the solid phase and then add the binding buffer. Preferably, the binding buffer is mixed with the sample to provide the binding mixture.

At the end of step (a), the DNA molecules having a size above the cut-off value are bound to the solid phase having an unmodified silica surface.

In step (b), the DNA molecules that are bound to the solid phase are separated from the remaining sample. Thereby, the bound larger DNA molecules having a size above the cut-off value are separated from unbound DNA molecules present in the sample. Suitable separation methods are well known in the prior art and the appropriate separation technique also depends on the used solid phase. E.g. when using a column based approach, separation is usually achieved when the binding mixture passes through the column. The DNA molecules having a size above the cut-off value bind to the solid phase comprised in the column while the remaining sample passes through the column and thereby is separated from the bound DNA molecules. As described above, this process can be assisted e.g. by centrifugation or by applying a vacuum. When using a particulate solid phase such as e.g. silica particles, the particles can be collected by sedimentation which can be assisted by centrifugation or magnetic separation if magnetic particles are used and the supernatant is separated off (e.g. decanted or aspirated or the particles are taken out of the liquid). Suitable embodiments were described above in conjunction with the different formats of the solid phase and their processing and are also well-known to the skilled person.

The separated DNA molecules can either be discarded in case the bound DNA is not of interest and shall merely be removed or can be further processed in case it represents the target DNA. The various uses and options were already described above and are also described in further detail below.

The bound DNA may optionally be washed. Here, one or more washing steps can be performed. Even though this step is optional, it is preferably performed in order to efficiently remove unbound components and impurities such as e.g. nucleotides and enzymes from previous reactions in case the bound DNA represents the target DNA. This is particularly suitable if the DNA containing sample was obtained during the preparation of a sequencing library. Furthermore, washing steps are also suitable to remove traces of the poly(alkylene oxide) polymer, the divalent cation and optionally salts if used during binding. Thus, according to one embodiment, one or more washing steps are performed in order to further purify the bound DNA molecules. For this purpose, common suitable washing solutions may be used. A suitable washing solution removes impurities but not the target DNA that is bound to the solid phase. According to one embodiment, the solution used for washing comprises at least one alcohol. As alcohol, short chained branched or unbranched alcohols with preferably one to 5 carbon atoms can be used for washing, respectively can be used in the washing solution. Also mixtures of alcohols can be used. Suitable alcohols include but are not limited to methanol, ethanol, propanol, isopropanol and butanol. Preferably, isopropanol and/or ethanol are used in the washing solution. A further suitable washing solution which can be used alternatively or also in addition to the washing solutions described above comprises an alcohol and a buffering agent. Suitable alcohols and buffering agents such as biological buffers are described above. Preferably, isopropanol or ethanol, most preferred ethanol is used for this second washing step. Preferably, ethanol is used in a concentration of at least 60% (v/v), at least 70% (v/v), preferably at least 80% (v/v). The buffering agent may preferably be Tris e.g. at a pH of approx. 7 to 8. A further suitable washing solution which can be used alternatively or optionally also in addition to the washing solutions described above comprises an alcohol but no salt. This allows to wash away salts. Preferably, isopropanol or ethanol, most preferred ethanol is used for washing. Preferably, the alcohol is comprised in a concentration of at least 50% v/v, at least 60% v/v, preferably at least 70% v/v. Preferably, the concentration lies in a range of 50% v/v to 100%v/v, more preferred 70% v/v to 100% v/v.

Residual alcohol that may be present after the washing step in case an alcohol containing washing solution was used can be removed e.g. by air drying (e.g. suitable when working with a particulate solid phase) or by an additional centrifugation step if using a column based solid phase. Respective methods and procedures are well-known in the prior art and thus, do not need any further description here.

Moreover, one or more elution steps are performed in order to elute the size selected target DNA. However, the bound DNA molecules may also be processed while being bound to the solid phase, depending on the intended downstream application, respectively the intended use of the size selected DNA. E.g. a particulate solid phase such as magnetic particles may be directly subjected to an amplification reaction without prior separate elution of the bound DNA.

However, it is preferred to elute the DNA. Here, basically any elution solution can be used which effects desorption of the bound DNA from the solid phase. Classical elution solutions known to effectively elute DNA from a silica surface include but are not limited to water (e.g. deionized water), elution buffers such as TE-buffer and low-salt solutions which have a salt content of 150mM or less, preferably 100mM or less, more preferred 75mM or less, 50mM or less, 25mM or less, 20mM or less, 15mM or less, 10mM or less or are salt-free. The elution solution may e.g. comprise a buffering agent, in particular may comprise a biological buffer such as Tris, MOPS, HEPES, MES, BIS-TRIS, propane and others. The buffering agent may be present in a concentration of 150mM or less, preferably 100mM or less, more preferred 75mM or less, 50mM or less, 25mM or less, 20mM or less, 15mM or less or 10mM or less. According to one embodiment, the elution buffer has a pH value that is selected from pH 6.5 to pH 10, pH 7 to pH 9.5, pH 7.5 to 9.0, pH 7.75 to pH 8.75 and pH 8 to pH 8.6. Elution can be assisted by heating and/or shaking what is e.g. particularly feasible if a particulate solid phase is used. If a column based solid phase is used, the elution buffer is usually applied to the column and forced through the solid phase which can be assisted again e.g. by centrifugation or by applying pressure or a vacuum.

Preferably, an elution solution is used that does not interfere with the intended downstream application. Thus, according to one embodiment, the elution buffer does not comprise a complexing agent such as EDTA. EDTA, in particular in higher concentrations, may inhibit downstream reactions.

Furthermore, it is also within the scope of the present invention to repeat the elution step in order to ensure that the bound DNA is efficiently released from the solid phase.

The method for isolating DNA by size according to the present invention is precise and reproducible with respect to the size of the isolated DNA molecules and leads due to the used alkaline pH value and the divalent cation in the binding mixture to high DNA recovery rates even though a solid phase having an unmodified silicon containing surface is used. Thus, in contrast to prior art methods, no expensive surface modified solid phases are required. The method according to the present invention is suitable for batch procedures. This e.g. distinguishes the methods of the present invention from prior art chromatographic methods which were used for size separation of nucleic acids, e.g. using titratable anion exchange compositions. The methods of the present invention in particular do not need highly specific and expensive apparatuses, do not need the generation and use of binding and/or elution buffer gradients and the collection and screening of multitudes of elution fractions. Therefore, according to one embodiment, no pH gradients are used in the method according to the present invention. The method is advantageously suitable for automation. Here, it is favourable to use magnetic particles for providing the solid phase.

Non-limiting preferred embodiments and applications of the method according to the present invention will be described further in the following. As discussed above, the size selective DNA isolation method according to the present invention is in particular suitable for enriching DNA molecules having a desired size above a certain cut-off value and/or having a size that lies within a certain size range (determined by an upper and lower cut-off value) from a mixed population of DNA molecules having different lengths. The method is in particular suitable for removing non-target DNA molecules which have a size below a certain cut-off value by binding and thus isolating target DNA molecules having a desired minimum size above the cut-off value from the DNA containing sample. In addition, removal of non-target molecules above a certain cut-off is possible, thus improving e.g. the heterogeneity of a DNA library and removing undesired artifacts such as PCR repeats.

According to one embodiment, the method is performed as follows:
(a) preparing a binding mixture comprising the DNA containing sample, a polyethylene glycol in a concentration that lies in the range of 7.5% to 15%, preferably 8% to 13%, at least one alkali metal salt and MgCl₂ in a concentration that lies in the range of 7mM to 40mM, preferably 8mM to 30mM, more preferably 8mM to 20mM, wherein said binding mixture has a pH that lies in the range of 8.5 to 9.25 and binding precipitated DNA molecules to a solid phase having an unmodified silicon containing surface, thereby providing a solid phase having bound thereto DNA molecules having a size above the cut-off value, wherein under the used binding conditions DNA molecules having a size which is less than the cut-off value predominantly do not bind to the solid phase;
(b) separating the bound DNA molecules from the remaining sample;
   - optionally washing the bound DNA molecules; and
   - optionally eluting the bound DNA molecules from the solid phase.

According to a preferred embodiment the method is used for isolating target DNA molecules having a size within a certain size range that is determined by an upper cut-off value and a lower cut-off value from a DNA containing sample, wherein said method comprises
(a) preparing a first binding mixture comprising the DNA containing sample, at least one poly(alkylene oxide) polymer which is a polyethylene glycol, at least one alkali metal salt and at least one divalent cation, wherein the first binding mixture comprises the divalent cation in a concentration of 3mM to 75mM, and wherein said first binding mixture has a pH that lies in the range of 8.5 to 9.5 and binding precipitated DNA molecules to a solid phase having an unmodified silicon containing surface, thereby providing a solid phase having bound thereto DNA molecules having a size above the upper cut-off value, wherein under the used binding conditions target DNA molecules having a size which is less than the upper cut-off value predominantly do not bind to the solid phase;
(b) separating the bound DNA molecules from the remaining sample which comprises the target DNA molecules;
(c) preparing a second binding mixture comprising the remaining sample, wherein said second binding mixture has a pH that lies in the range of 8.5 to 9.5 and wherein the concentration of the poly(alkylene oxide) polymer which is a polyethylene glycol in the second binding mixture is increased compared to the first binding mixture and binding precipitated target DNA molecules to a solid phase having an unmodified silicon containing surface, thereby providing a solid phase having bound thereto target DNA molecules having a size above the lower cut-off value, wherein under the used binding conditions DNA molecules having a size which is less than the lower cut-off value predominantly do not bind to the solid phase;
(d) separating the bound target DNA molecules from the remaining sample;
   - optionally washing the bound target DNA molecules; and
   - optionally eluting the bound target DNA molecules from the solid phase.

This advantageously allows to isolate target DNA molecules within a defined size range. Suitable concentrations for the poly(alkylene oxide) polymer which is a polyethylene glycol for the first and second binding step were described above. As discussed herein, it is preferred to adjust the binding conditions in the first and second binding mixture using a binding buffer as described above. According to one embodiment, the same binding buffer is used for preparing the first and second binding mixture. The concentration of the poly(alkylene oxide) polymer which is a polyethylene glycol in the binding mixture can e.g. be varied and adjusted by adding different volumes of the binding buffer.

According to one embodiment, the method is performed as follows:
(a) preparing a first binding mixture comprising the DNA containing sample, polyethylene glycol in a concentration that lies in the range of 8.5% to 9.5%, at least one alkali metal salt and MgCl₂ in a concentration that lies in the range of 7mM to 40mM, preferably 8mM to 30mM, more preferably 8mM to 20mM, wherein said binding mixture has a pH that lies in the range of 8.5 to 9.25 and binding precipitated DNA molecules to a solid phase having an unmodified silicon containing surface, thereby providing a solid phase having bound thereto DNA molecules having a size above the upper cut-off value, wherein under the used binding conditions target DNA molecules having a size which is less than the upper cut-off value predominantly do not bind to the solid phase;
(b) separating the bound DNA molecules from the remaining sample which comprises the target DNA molecules;
(c) preparing a second binding mixture comprising the remaining sample, wherein said second binding mixture has a pH that lies in the range of 8.5 to 9.25 and wherein the concentration of polyethylene glycol in the second binding mixture is increased compared to the first binding mixture and lies in a range of 10% to 12% and binding precipitated target DNA molecules to a solid phase having an unmodified silicon containing surface, thereby providing a solid phase having bound thereto target DNA molecules, wherein under the used binding conditions DNA molecules having a size which is less than the lower cut-off value predominantly do not bind to the solid phase;
(d) separating the bound target DNA molecules from the remaining sample;
   - optionally washing the bound target DNA molecules; and
   - optionally eluting the bound target DNA molecules from the solid phase.

As discussed above, the method according to the present invention is in particular suitable for size selection in the context of next generation sequencing. The preparation of a sequencing library suitable for next generation sequencing usually is a multi-step process wherein at different stages of said process a size-selection of the provided DNA molecules may be performed. At which stage of said process a size selection is performed also depends on the library preparation method used. Suitable embodiments will be described in the following. The size selection method according to the present invention is in particular suitable for use in the context of preparing a sequencing library, as it allows the separation of DNA fragments with only small differences in size, e.g. as described herein, the removal of unwanted adapter dimers (approx. 120bp) from the desired DNA fragments (150bp and larger) in library construction protocols for next generation sequencing applications.

A sequencing library which is suitable for massive parallel sequencing and accordingly, is suitable for next generation sequencing can be prepared using methods known in the prior art. The preparation of a respective sequencing library often involves the generation of a plurality of double-stranded, linear DNA fragments from a nucleic acid containing sample. For example, DNA, such a genomic DNA or cDNA, can be fragmented for example by shearing, such as sonification, hydro-shearing, ultrasound, nebulization or enzymatic fragmentation, in order to provide DNA fragments that are suitable for subsequent sequencing. The length of the fragments can be chosen based on the sequencing capacity of the next generation sequencing platform that is subsequently used for sequencing. Usually, the obtained fragments have a length of 1500bp or less, 1000bp or less, 750bp or less, 600bp or less and preferably 500bp or less as this corresponds to the sequencing capacity of most current next generation sequencing platforms. Preferably, the obtained fragments have a length that predominantly lies in a range of 50bp to 1000bp, more preferred 75bp to 900bp, 100bp to 850bp, 110bp to 800bp, 115bp to 750bp, 120bp to 700bp, 125bp to 650bp, 130bp to 600bp, 135bp to 550bp, 140bp to 500bp and 145bp to 450bp. Respective fragment sizes are particularly suitable for genomic DNA, also considering that the size of an exon is approx. 150bp to 200bp in length and respective short fragments can be efficiently sequenced using common next generation sequencing platforms. However, also longer fragments can be useful, e.g. if using next generation sequencing methods which allow longer sequence reads.

According to one embodiment, the fragmented DNA is repaired after fragmentation and end polished using methods known in the prior art, thereby providing DNA fragments having blunt ends. In such methods which are well-known in the prior art, overhangs resulting from the fragmentation process are converted into blunt ends. As the respective methods are well-known in the prior art, they do not need any detailed description herein.

According to one embodiment, the size selective DNA isolation method according to the invention is performed after DNA fragments were obtained, preferably after the fragmented DNA was end polished to provide DNA fragments having blunt ends. A size selective DNA isolation at this stage allows e.g. to eliminate very short DNA fragments which do not have the appropriate length for subsequent sequencing. As discussed above, the cut-off value for DNA binding can be adjusted by appropriate choice of the concentration of the poly(alkylene oxide) polymer in the binding mixture. The present method allows to isolate DNA fragments that substantially have a size within a defined size range.

According to one embodiment, after end-repair and optionally size selection, an overhang is added to the 3' ends of the blunt end fragments. Preferably, a single nucleotide overhang is added. E.g. a single "A" nucleotide can be added using methods well-known in the prior art. This is also referred to as "A tailing". A respective nucleotide overhang prevents the fragments from ligating to one another during the subsequent adapter ligation reaction. E.g. a corresponding single "T" or other complementary overhang can be provided at the 3' end of the adapters to provide a complementary overhang for ligating the adaptors to the DNA fragment. This ensures a low rate of chimera (concatenated template) formation. However, also other strategies are known in the prior art to ensure proper ligation of sequencing adapters. E.g. also blunt end adapters can be ligated.

According to a preferred embodiment, adapters are ligated to the 5' and/or 3' ends of the obtained DNA fragments, preferably at both ends of the DNA fragments. The specific design of the adapters depends on the next generation sequencing platform to be used and for the purposes of the present invention, basically any adaptors used for preparing sequencing libraries for next generation sequencing can be used. The adapter sequences provide a known sequence composition allowing e.g. subsequent library amplification and/or sequencing primer annealing. As adaptors, double-stranded or partially double-stranded nucleic acids of known sequence can be used. The adapters may have blunt ends, cohesive ends with 3' or 5'overhangs, may be provided by Y shaped adapters or by stem-loop shaped adapters. Y shaped adapters are e.g. described in US7,741,463 and stem-loop shaped adapters are e.g. described in US2009/0298075, herein incorporated by reference regarding the specific design of the adapters. Preferably, the adaptors have a length of at least 7, preferably at least 10, preferably at least 15 bases. The adapter length preferably lies in a range of 10 to 100 bases, preferably 15 to 75 bases, more preferred 20 to 60 bases. Either the same or different adapters can be used for the 3' and 5' end of the DNA fragments. Using the same type of adaptor for both ends, such as e.g. a Y shaped or a stem-looped shaped adapter, has the advantage that no fragments are lost during library preparation due to adapter mispairing which is an advantage when working with low amounts of DNA.

Thus, according to one embodiment, a sequencing library is prepared which comprises randomly fragmented double stranded DNA molecules which are ligated at their 3' and 5' end to adapter sequences. The adaptors provide a known sequence and thus provide a known template for amplification and/or sequencing primers. Optionally, the adapters may also provide an individual index thereby allowing the subsequent pooling of two or more target enriched sequencing libraries prior to sequencing. This embodiment will be described in further detail below.

To ensure an efficient adapter ligation, the adapters are usually used in excess during the adapter ligation step. Thus, after adapter ligation, a DNA containing sample is provided which comprises DNA molecules that are flanked by adapters in addition to unligated adapter monomers and adapter-adapter ligation products such as adapter dimers. Unligated adapter monomers and adapter-adapter ligation products are usually comprised in large amounts in the sample that is obtained after the adapter ligation process. It is important to remove these unligated adapter monomers and adapter-adapter ligation products as they otherwise diminish the sequencing power of the subsequent sequencing reaction. To remove unligated adapter monomers and adapter-adapter ligation products as well as enzymes and other contaminants from the adapter ligation sample, it is preferred to perform a size selective DNA isolation using the method according to the present invention. The cut-off value for DNA is chosen such that it lies above the size of unligated adapter monomers and above the size of expected adapter-adapter ligation products. This ensures that unligated adapter monomers and unwanted adapter-adapter ligation products are not captured during said size selective DNA isolation step and thus are depleted from the isolated target DNA, which predominantly comprises DNA molecules having a size above the cut-off value. The method according to the present invention is particularly advantageous for this purpose, because the cut-off value can be precisely adjusted within a narrow range as is demonstrated by the examples by varying the concentration of the poly(alkylene oxide) polymer in the binding mixture.

Thus, according to one embodiment, the method of the invention is used for isolating adapter ligated DNA molecules as target DNA molecules from a DNA containing sample which is an adapter ligation sample and for removing adapter monomers and adapter-adapter ligation products, wherein adapter ligated DNA molecules are separated from unligated adapter monomers and adapter-adapter ligation products based on the larger size of the adapter ligated DNA molecules. Said method may comprise
(a) preparing a binding mixture comprising the adapter ligation sample, at least one poly(alkylene oxide) polymer which is a polyethylene glycol, at least one alkali metal salt and at least one divalent cation, wherein the binding mixture comprises the divalent cation in a concentration of 3mM to 75mM, and wherein said binding mixture has a pH that lies in the range of 8.5 to 9.5 and binding precipitated adapter ligated DNA molecules to a solid phase having an unmodified silicon containing surface, thereby producing a solid phase having bound thereto the adapter ligated DNA molecules, wherein under the used binding conditions adapter monomers and adapter-adapter ligation products predominantly do not bind to the solid phase;
(b) separating the bound adapter ligated DNA molecules from the remaining sample;
   - optionally washing the bound adapter ligated DNA molecules; and
   - optionally eluting the bound adapter ligated DNA molecules from the solid phase.

The cut-off value lies above the size of adapter monomers and above the size of expected adapter-adapter ligation products. According to one embodiment, the cut-off value lies at least 10nt, at least 15nt, at least 20nt, at least 25nt or at least 30nt above the size of expected adapter-adapter ligation product(s). Furthermore, double-size selection can be performed as described above using an adapter ligation sample as DNA containing sample.

According to one embodiment, target DNA fragments are enriched after adapter ligation (and preferably size selection as described above) by amplification, preferably PCR amplification. Such enrichment step is optional, but preferred for some applications. E.g. an amplification reaction such as a PCR amplification can be used to selectively enrich those DNA fragments that have adapter molecules on both ends and to amplify the amount of DNA in the library. According to one embodiment, the PCR is performed with one or more primers that anneal to the adapters. Respective amplification steps are well known in the prior art and thus, do not need any detailed description here. According to one embodiment, a size-selective DNA isolation according to the method of the present invention is performed after amplification. The cut-off value is again chosen such that primers, unligated adapter monomers and adapter-adapter ligation products that might have been present during amplification and might have been amplified, are not captured during DNA binding. According to one embodiment, the same cut-off value is used as was used when performing a size selective DNA isolation after the adapter ligation step.

Thus, according to one embodiment, the method according to the present invention comprises amplifying the isolated, size selected adapter ligated double stranded DNA molecules to provide an enriched sequencing library, wherein after amplification, a size selection step is performed which comprises
(a) contacting the amplified sample with a binding buffer which comprises at least one poly(alkylene oxide) polymer which is a polyethylene glycol, at least one alkali metal salt, at least one divalent cation and at least one buffering agent, wherein the binding mixture comprises the divalent cation in a concentration of 3mM to 75mM, and wherein said binding buffer has a pH that lies in a range of 8.5 to 9.5 to provide a binding mixture and binding precipitated target DNA molecules having a size above the cut-off value to a solid phase having an unmodified silicon containing surface;
(b) separating the bound target DNA from the remaining sample;
   - optionally washing the bound target DNA; and
   - optionally eluting the bound target DNA from the solid phase.

After amplification enrichment, which preferably is followed by a size selective DNA isolation according to the present invention, the sequencing library is ready for use. Optionally, the prepared sequencing library can be validated, quantified and/or quality controls can be performed to verify the size of the obtained adapter ligated fragments, respectively PCR enriched fragments.

Suitable general methods for preparing sequencing libraries are also described in Metzker, 2011, Voelkerding, 2009, and WO12/003374 and can be combined with the size selective DNA isolation method according to the present invention.

According to one embodiment, the present invention provides a method for preparing a sequencing library that is suitable for massive parallel sequencing, wherein said method comprises
A) fragmenting DNA and optionally end repairing DNA fragments to provide a sample comprising blunt end DNA fragments of different sizes;
B) optionally performing a step of isolating target DNA having a fragment size above a certain cut-off value, wherein said optional size selection step comprises
   (a) contacting the sample comprising the DNA fragments of different sizes with a binding buffer which has a pH that lies in a range of 8.5 to 9.5 and which comprises at least one poly(alkylene oxide) polymer which is a polyethylene glycol, at least one alkali metal salt, at least one divalent cation and at least one buffering agent to provide a corresponding binding mixture, wherein the binding mixture comprises the divalent cation in a concentration of 3mM to 75mM, and binding precipitated target DNA molecules having a size above the cut-off value to a solid phase having an unmodified silicon containing surface;
   (b) separating the bound target DNA from the remaining sample;
      - washing the bound target DNA; and
      - eluting the bound target DNA from the solid phase;
C) performing an adapter ligation step to provide a sample comprising double-stranded DNA molecules that are flanked 5' and/or 3' by adapters,
D) isolating and thus separating adapter ligated double-stranded DNA molecules from unligated adapter monomers and adapter-adapter ligation products based on the larger size of the adapter ligated double stranded DNA molecules wherein said size selection step comprises
   (a) preparing a binding mixture comprising the adapter ligation sample, at least one poly(alkylene oxide) polymer which is a polyethylene glycol, at least one alkali metal salt and at least one divalent cation, wherein the binding mixture comprises the divalent cation in a concentration of 3mM to 75mM, and wherein said binding mixture has a pH that lies in the range of 8.5 to 9.5 and binding adapter ligated DNA molecules that were precipitated from said binding mixture to a solid phase having an unmodified silicon containing surface, thereby producing a solid phase having bound thereto the adapter ligated DNA molecules, wherein under the used binding conditions adapter monomers and adapter-adapter ligation products predominantly do not bind to the solid phase;
   (b) separating the bound adapter ligated DNA molecules from the remaining sample;
      - washing the bound adapter ligated DNA molecules; and
      - eluting the bound adapter ligated DNA molecules from the solid phase;
E) optionally amplifying adapter ligated DNA molecules.

This embodiment provides a sequencing library comprising adapter ligated DNA fragments having an appropriate minimal length. As discussed above, preferably, adapters are provided at the 3' end and the 5' end of the DNA fragments. Furthermore, the size selection step performed in step D) efficiently removes adapter monomers and adapter-adapter ligation products as well as other contaminants from the ligation reaction. This improves the quality of the sequencing library. Step D) can also be repeated and thus be performed two or more times in order to ensure an efficient removal of substantially all adapter monomers and adapter-adapter ligation products and to increase the size selection stringency. The method is fast, reliable and provides a sequencing library of high quality. Furthermore, said method can be easily integrated into existing sequencing library preparation methods. The described method can performed mutatis mutandis using the embodiment, wherein size selection occurs for an upper and lower cut-off value to provide target DNA molecules having a size within a certain size range (see claim 2).

A single NGS run usually produces enough reads to sequence several target enriched sequencing libraries at once. Therefore, pooling strategies and indexing approaches are a practical way to reduce the per sample cost. Respective multiplexing strategies can also be used in conjunction with the teaching of the present invention. Features enabling multiplexing can be incorporated in different stages of the enrichment process. According to one embodiment, the sequencing library is generated by using adaptors containing specific sequence motifs for library labelling and differentiation ("barcoded" or "index" adapters). Each sequencing library is provided with individual und thus library specific adapters which provide a library specific sequence. Preferably, each adapter comprises besides the index region a common universal region which provides a known template for PCR primers and/or sequencing primers that can be used on all libraries. After the target enriched sequencing libraries were obtained, they can be pooled and sequenced in a single run. Providing the DNA fragments of the sequencing library with respective index adaptors thus allows subsequently sequencing several target enriched sequencing libraries in the same sequencing run because the sequenced fragments can be distinguished based on the library specific sequence of the index adaptors. After sequencing, the individual sequences belonging to each library can be sorted via the library specific index which is then found in the obtained sequence. Respective index approaches are known in the prior art and index adapters are also commercially available and are for example provided in the TruSeq^{®} DNA sample prep kits which are suitable for use in the Illumina platform.

According to one embodiment, the sequencing library comprises the double-stranded DNA molecules in an overall amount of 3 µg or less, 2 µg or less, 1.5 µg or less, 1 µg or less, 0.75 µg or less or 0.5 µg or less. According to one embodiment, the sequencing library comprises the double-stranded DNA molecules in an overall amount of at least 0.2µg, at least 0.5µg or at least 0.75µg. The method according to the resent invention not only enables a size-selective DNA isolation but also ensures an efficient capture of DNA molecules having the desired size. This is an important advantage, because in many cases, the sequencing library comprises the DNA in low amounts as DNA material might also get lost during the preparation of the sequencing library.

Nucleic acids such as DNA and/or RNA can be isolated from a sample of interest according to methods known in the prior art to provide the starting material for preparing the sequencing library. RNA is usually first transcribed into cDNA prior to preparing the sequencing library.

As discussed above, sequencing is preferably performed on a next generation sequencing platform. All NGS platforms share a common technological feature, namely the massively parallel sequencing e.g. of clonally amplified or single DNA or cDNA molecules that are spatially separated in a flow cell or by generation of an oil-water emulsion. In NGS, sequencing is performed by repeated cycles of polymerase-mediated nucleotide extensions or, in one common format, by iterative cycles of oligonucleotide ligation. After obtaining the sequencing library using the method according to the present invention, clonal separation of single molecules and subsequent amplification is performed by in vitro template preparation reactions like emulsion PCR (pyrosequencing from Roche 454, semiconductor sequencing from Ion Torrent, SOLiD sequencing by ligation from Life Technologies, sequencing by synthesis from Intelligent Biosystems), bridge amplification on the flow cell (e.g. Solexa/lllumin(a), isothermal amplification by Wildfire technology (Life Technologies) or rolonies/nanoballs generated by rolling circle amplification (Complete Genomics, Intelligent Biosystems, Polonator). Sequencing technologies like Heliscope (Helicos), SMRT technology (Pacific Biosciences) or nanopore sequencing (Oxford Nanopore) allow direct sequencing of single molecules without prior clonal amplification. Suitable NGS methods and platforms that can be used were also described in the background of the present invention and it is referred to the respective disclosure. The sequencing can be performed on any of the respective platforms. According to one embodiment, wherein the method of the present invention is used in order to remove unligated adapter monomers or adapter dimers, after sequencing the respectively obtained sequencing library, the reads for adapter-dimers relative to the number of total reads is ≤ 1.5%, preferably ≤ 1.25%, ≤ 1%, ≤ 0.75%, ≤ 0.6%, more preferred ≤ 0.5%, ≤ 0.4%, ≤ 0.3%, more preferred ≤ 0.2% and most preferred ≤ 0.15%.

According to one embodiment, the method according to the present invention is performed more than once. Thus, according to one embodiment, at least two size selective DNA isolation cycles using the method of the present invention comprising steps (a) and (b) are performed. According to one embodiment, an eluate that is obtained in the first size selection cycle provides the DNA containing sample for the second size selection cycle. Thus, the eluate obtained after the first size selection cycle is contacted in the second size selection cycle in step (a) with the binding buffer in order to provide a binding mixture and to bind target DNA molecules having a size above the desired cut-off value to the solid phase having an unmodified silicon containing surface. According to one embodiment, the same binding conditions are used in the second size selection cycle as in the first size selection cycle. According to another embodiment, different binding conditions are used in the second size selection cycle, e.g. using a different concentration of the at least one poly(alkylene oxide) polymer which is a polyethylene glycol in the binding mixture in order to adjust a different cut-off value. The same type of solid phase having an unmodified silicon containing surface can be used in the first and second size selection cycle. Performing at least two size selection cycles according to the present invention can be favourable to remove large amounts of contaminating short DNA fragments such as e.g. adapter monomers or adapter-adapter ligation products during the preparation of a sequencing library, in particular a sequencing library suitable for next generation sequencing.

According to one embodiment, the method according to the present invention is used for fractionating DNA molecules comprised in a DNA containing sample according to their size. Thereby, two or more fractions are obtained, wherein the DNA molecules comprised in the different fractions differ on average in their length. The method according to the present invention is also suitable for said purpose. For fractionating a DNA containing sample, e.g. two or more size selective DNA isolation cycles are performed. Size fractionation can be e.g. achieved as follows:
- in the first size selective DNA isolation cycle A binding conditions A are provided in the binding mixture which determines the cut-off value A, and wherein the obtained eluate A provides a fraction which predominantly comprises target DNA molecules having a size above the cut-off value A;
- wherein the separated remaining sample obtained in step (b) of the first size selective DNA isolation cycle A provides the DNA containing sample for the second size selective DNA isolation cycle B, and wherein binding conditions B are provided in the binding mixture which determines the cut-off value B, wherein the cut-off value B is lower than the cut-off value A and wherein the obtained eluate B provides a fraction which predominantly comprises target DNA molecules having a size above the cut-off value B but below the cut-off value A.

Details regarding suitable binding conditions were described already above and it is referred to the respective disclosure. According to one embodiment, the poly(alkylene oxide) polymer which is a polyethylene glycol is present in the first binding mixture in a concentration that lies in the range of 8.5% to 9.5% (upper cut-off value A) and is present in the second binding mixture in a concentration of 10% to 12%, preferably 10.5 to 11.5% (lower cut-off value B). The binding conditions can be adjusted by adding different volumes of the same binding buffer. If desired, the DNA molecules having a size smaller than the cut-off value B that are still comprised in the remaining sample can be subjected to a third size selective DNA isolation cycle C, if desired. Thus, according to one embodiment, a size selective DNA isolation cycle C is performed, wherein the separated remaining sample obtained in step (b) of the second size selective DNA isolation cycle B provides the DNA containing sample for the third size selective DNA isolation cycle C, and wherein binding conditions C are provided in the binding mixture which determines the cut-off value C, wherein the cut-off value C is smaller than the cut-off value B and wherein the obtained eluate C provides a fraction which predominantly comprises DNA molecules having a size above the cut-off value C but below the cut-off value B.

### KIT

According to a second aspect, a kit is provided for the size selective isolation of target DNA molecules having a size above a desired cut-off value from a DNA containing sample, wherein the method is suitable for the method according to the first aspect, the kit comprising
(a) a binding buffer comprising at least one poly(alkylene oxide) polymer which is a polyethylene glycol, at least one alkali metal salt, at least one divalent cation and at least one buffering agent wherein the binding buffer has a pH value that lies in the range of 8.5 to 9.5;
(b) a solid phase having an unmodified silicon containing surface;
(c) optionally at least one washing solution; and
(d) optionally at least one elution solution.

Details regarding the binding buffer, in particular suitable and preferred binding buffer components, binding buffer component concentrations and pH values, as well as details regarding the solid phase, the washing solution and the elution solution were described in detail above in conjunction with the method according to the present invention. It is referred to the above disclosure which also applies here. Non-limiting selected embodiments are again described subsequently.

Suitable and preferred concentrations for the poly(alkylene oxide) polymer which is a polyethylene glycol in the binding buffer were described above and it is referred to the above disclosure. Preferably, the concentration of the at least one poly(alkylene oxide) polymer which is a polyethylene glycol in the binding buffer is selected from 7% to 45%, 10% to 40%, 12.5% to 35%, 15% to 30%, 17.5% to 27.5% and 20% to 25%. Preferably, the poly(alkylene oxide) polymer is unsubstituted polyethylene glycol.

The binding buffer preferably has a pH value that lies in the range of 8.6 to 9.2.

Suitable buffering agents and concentrations in the binding buffer were described above and it is referred to the above disclosure which also applies here. The binding buffer may comprise a buffering agent in a concentration selected from 25mM to 1M, 50mM to 750mM, 75mM to 700mM, 100mM to 650mM, 125mM to 600mM, 150mM to 550mM, 175mM to 525mM and 200mM to 500mM.

The binding buffer comprises an alkali metal salt, preferably an alkali metal halide. The salt is preferably selected from sodium chloride, potassium chloride, lithium chloride and cesium chloride, more preferably the salt is sodium chloride. Salts were also described in conjunction with the method and it is referred to the respective disclosure. The concentration of the salt may be selected from 0.5M to 4M, 0.7M to 3.5M, 1M to 3M, 1.2M to 2.75M and 1.3M to 2.5M.

Suitable and preferred embodiments of the solid phase were also described in conjunction with the method according to the invention. As described above, the solid phase preferably provides an unmodified silica surface.

According to one embodiment, the binding buffer has the following features:
i) it comprises Mg²⁺ as divalent cation, preferably as MgCl₂;
ii) it comprises polyethylene glycol in a concentration selected from 12.5% to 35%, 15% to 30%, 17.5% to 27.5% and 20% to 25%;
iii) it comprises an alkali metal salt, preferably sodium chloride or potassium chloride, more preferably sodium chloride, in a concentration that lies in the range of 0.5M to 2.75M, preferably 0.75M to 2.5M;
iv) it comprises the at least one buffering agent in a concentration that lies in a range of 100 to 550mM; and
v) it has a pH that lies in the range of 8.6 to 9.2.

Furthermore, the kit may comprise instructions and/or information for use. E.g. the kit may comprise instructions and/or information regarding the cut-off value that is achieved when mixing a certain volume of the binding buffer with a certain volume of the DNA containing sample and/or the cut-off value(s) that are achieved if the DNA containing sample is mixed in a certain ratio with the binding buffer. If two or more binding buffers are comprised in the kit that differ in the concentration of the poly(alkylene oxide) polymer which is a polyethylene glycol, the kit may provide information which cut-off value is achieved when using a certain binding buffer comprised in the kit. Thus, the present invention also provides a kit which allows the flexible adjustment of the cut-off value e.g. by mixing a certain volume of the binding buffer and a certain volume of the DNA containing sample.

A respective kit can be in particular used in the method according to the first aspect. In particular, it can be used for fractionating DNA molecules comprised in a DNA containing sample according to their length.

Numeric ranges are inclusive of the numbers defining the range. The headings provided herein are not limitations of the various aspects or embodiments of this invention which can be read by reference to the specification as a whole.

The term "solution" as used herein in particular refers to a liquid composition, preferably an aqueous composition. It may be a homogenous mixture of only one phase but it is also within the scope of the present invention that a solution comprises solid constituents such as e.g. precipitates.

As used in the subject specification and claims, the singular forms "a", "an" and "the" include plural aspects unless the context clearly dictates otherwise. Thus, for example, reference to "a poly(alkylene oxide) polymer" includes a single type of poly(alkylene oxide) polymer, as well as two or more poly(alkylene oxide) polymers. Likewise, reference to "a divalent cation", "an additional salt", "a buffering agent" and the like includes single entities and combinations of two or more of such entities. Reference to "the disclosure" and "the invention" and the like includes single or multiple aspects taught herein; and so forth. Aspects taught herein are encompassed by the term "invention".

The solid phase is not considered when determining the concentrations of the components, such as the poly(alkylene oxide) polymer, the divalent cation, the salt or the buffering agent in the binding mixture.

According to one embodiment, subject matter described herein as comprising certain steps in the case of methods or as comprising certain ingredients in the case of compositions, solutions and/or buffers refers to subject matter consisting of the respective steps or ingredients. It is preferred to select and combine preferred embodiments described herein and the specific subject-matter arising from a respective combination of preferred embodiments also belongs to the present disclosure.

### EXAMPLES

The invention is illustrated by the following non-limiting examples.

### 1. Example 1

Binding/precipitation buffer stock solution: 22% PEG 8000, 2M NaCl, 200mM Tris either with or without 20 mM MgCl₂. The pH of these precipitation buffers was adjusted to either pH 6 or 9 as is also indicated in Fig. 1. This precipitation buffer stock solution was contacted with sheared human DNA as DNA containing sample. DNA was sheared with the Covaris ultrasonicator in TE buffer to provide a DNA containing sample. 90µl of this DNA containing sample was mixed with 90µl precipitation buffer stock solution (concentration in the binding mixture: 11% PEG 8000, 1M NaCl, 100mM Tris either with or without 10 mM MgCI2). The sheared DNA was precipitated onto 10µl of a suspension comprising magnetic silica particles (MagAttract G beads - QIAGEN). The DNA that was precipitated onto the magnetic silica particles was washed twice with 80% ethanol and eluted with 50µl elution buffer.

The obtained eluates were analyzed on an Agilent 7500 chip in order to analyze the cut-off values achieved with the different binding/precipitation buffers and the DNA yield. The results are shown in Fig. 1 (electropherogram). The large peaks shown at 50bp and 10380bp result from the calibration markers. The curves between the markers resemble the DNA fragments of different sizes that were isolated with the different binding buffers. Thus, the curve inbetween the calibration markers shows the size distribution of the isolated DNA fragments and the height of the curve indicates the obtained yield. Furthermore, the curve of the input DNA is shown.

Fig. 1 demonstrates that shifting the pH of the precipitation buffer to approx. pH 9 together with the addition of the divalent cation Mg²⁺ surprisingly enhances the yield of recovered DNA fragments when using a solid phase having an unmodified silicon containing surface. Therefore, the binding conditions used in the present invention enhance the recovery of polyethylenglycol (PEG) precipitated DNA fragments on unmodified silica surfaces thereby allowing to use such unmodified solid phases for size selective DNA isolation with a poly(alkylene oxide) polymer.

### 2. Example 2

Example 2 demonstrates that the cut-off value of recovered DNA fragments can be relocated and hence can be adjusted by adding different amounts of the binding and hence precipitation buffer of the present invention. The DNA containing sample was sheared, human DNA. The binding/precipitation buffer stock solution comprised 22% PEG 8000, 2 M NaCl, 20 mM MgCl₂, 200 mM Tris pH 8.88. This binding buffer was added to the DNA containing sample in an amount so that the following end concentrations of PEG 8000 were achieved in the binding mixture: 14%, 12%, 10% and 8%.

The sheared DNA was precipitated onto 5 µl MagAttract suspension G (magnetic silica particles). In advance, the MagAttract G beads were preincubated with a buffer containing 2M NaCl, 20 mM MgCl₂, 200 mM Tris, pH 8.89 in order to rebuffer the silica particles. The beads with the bound DNA were washed twice with 80% ethanol and eluted with 17 µl of an elution buffer. The samples were again analyzed on an Agilent DNA 7500 chip. The obtained electropherogram is shown in Fig. 2. As can be seen, there is a concentration dependent shift in the cut-off value that correlates with the polyethylene glycol concentration. Higher concentrations of polyethylene glycol led to the recovery of smaller DNA fragments. When reducing the concentration of polyethylene glycol in the binding mixture, the cut-off value increased in a concentration dependent matter. Therefore, by using precipitation buffers comprising different amounts of PEG or by including different amounts of the precipitation buffer in the binding mixture, the cut-off value can be precisely adjusted.

Example 2 therefore shows that by adding different amounts of the binding/precipitation buffer according to the invention to the DNA containing sample, DNA fragments of different lengths can be separated. The size selective separation method is particularly suitable for applications such as library preparation for next generation sequencing, where unwanted, small DNA fragments like adapters or large fragments like misprimed PCR products have to be removed, while library DNA has to be retained as efficiently as possible. Another advantage of the size selection method according to the present invention in applications like library preparation is the flexible relocation of the cut-off. The invention allows this flexible relocation and hence adaption of the cut-off value while maintaining the enhanced yield of recovered DNA molecules. All cut-off values can be relocated simply by adding the binding buffer in different amounts.

### 3. Example 3

Example 3 demonstrates that the high target DNA yields and the adaption of the cut-off value is also achieved when using silica spin columns as solid phase. The DNA containing sample was again sheared, human DNA. The precipitation buffer stock solution concentration was 22% Peg 8000, 2 M NaCl, 20 mM MgCl₂, 200 mM Tris pH 8.88. This binding/precipitation buffer was added to the DNA containing sample so that the following end concentrations of PEG 8000 were achieved in the binding mixture: 9%, 8%, 7%. The sheared DNA was precipitated onto MinElute spin columns which were in advance pre-incubated with a buffer containing 2 M NaCl, 20 mM MgCl₂, 200 mM Tris, pH 8.89. The precipitated DNA that was bound to the contained silica membrane was washed twice with 80% ethanol and eluted in 17% µl elution buffer. The eluate was analyzed on an Agilent DNA 7500 chip. The electropherogram is shown in Fig. 3. As can be seen, the same polyethylene glycol concentration dependent relocation of the cut-off value is achieved. Furthermore, the recovery of DNA fragments was high.

### 4. Example 4

In example 4, the method of the invention was performed for isolating target DNA molecules having a size within a certain size range that is determined by an upper cut-off value and a lower cut-off value from a DNA containing sample. The results were compared with the results obtained with the reference AMPure XP according to the manufacturer's instructions. In brief, the AMpure XP Bead protocol was performed as follows: AMpure XP Beads were resuspended. 80 µl or 20 µl of beads were added to 100 µl of sample or to the supernatant in the microtiter plate. The pipette was adjusted to 160 µl and the entire volume was pipetted 10 times up and down for thorough mixture. The pipette tip was changed after each sample. The microtiter plate was incubated at room temperature for 15 minutes. The micro titer plate was put on a magnet plate and was left sitting at room temperature for 15 minutes or until the fluid appeared clear. Then, about half of each supernatant was removed and discarded and care are was taken not to touch the beads; this step was performed twice. The pipette tip was changed after each sample and there may be residual liquid in the well. While the microtiter plate is on the magnet plate, 200 µl of freshly prepared 80% ethanol were added to each sample and care was taken not to resuspended the beads. The microtiter plate was incubated at room temperature for at least 30 seconds. Afterwards, the complete supernatant was removed from each well and discarded. Care was taken not to touch the beads. The pipette tip was changed after each sample. The washing with ethanol was repeated. The microtiter plate was taken of the magnet plate and was left sitting for 15 minutes at room temperature in order to dry. The dried pellet was resuspended in 17.5 µl of resuspension buffer, here EB, by pipetting the entire volume carefully up and down 10 times. The microtiter plate was incubated at room temperature for 2 minutes. The microtiter plate was left sitting on the magnet plate for 5 minutes or until the liquid appeared clear. 15 µl of the clear supernatant were transferred into a new tube. Tips were changed after each sample.

In example 4, multiple different binding buffers were furthermore tested in the method of the invention. If not stated otherwise in example 4, the DNA containing sample was sheared, human DNA and the method was performed as follows:
- Magnetic silica particles (MagAttract G beads, QIAGEN) were preincubated in a wash buffer (5µl beads + 10µl wash buffer, mixing by vortexing). The wash buffer had the same composition as the binding buffer, except that it contained no PEG. The magnetic silica particles were separated by the aid of a magnet and the wash buffer removed.
- The washed magnetic silica particles (5µl) were contacted with 63µl binding buffer and 90µl DNA containing sample (1µg). Thereby, a first binding mixture is provided (1 part DNA sample to 0.7 part binding buffer) that comprises approx. 9% PEG. The magnetic silica particles are not considered in the calculation of the concentration of components in the binding mixture. The first binding mixture was mixed by vortexing and incubated for 10min while shaking the first binding mixture in order to allow binding of DNA molecules having a size above the upper cut-off value to the magnetic particles.
- The magnetic particles with the bound DNA molecules were magnetically separated by placing the samples onto a magnetic rack. Thereby, larger DNA molecules having a size above the upper cut-off value were removed from the first binding mixture. The sample remainder was further processed.
- 5µl magnetic silica particles (washed as described above) were contacted with a further volume of the binding buffer (25.5µl) and 140ml of the sample remainder. Thereby, a second binding mixture was provided that comprises approx. 11% PEG. The magnetic silica particles are again not considered in the calculation of the concentration of components in the binding mixture. The second binding mixture was mixed by vortexing and incubated for 10min while shaking the second binding mixture in order to allow binding of target DNA molecules having a size above the lower cut-off value to the magnetic particles.
- The magnetic particles with the bound target DNA molecules were magnetically separated by placing the samples onto a magnetic rack. The supernatant was removed and discarded.
- The magnetic silica particles with the bound target DNA molecules were washed with 500µl 80% ethanol, 5min incubation under shaking at RT and magnetic separation for 1min on the magnetic rack. The supernatant was removed and discarded. The washing step was performed twice.
- The washed magnetic silica beads with the bound target DNA molecules were air-dried for 5min.
- A first elution step was performed by adding 17µl buffer TE (QIAGEN) and incubating for 5min at RT under shaking. The suspension was placed onto a magnetic rack for separation and 15µl of the obtained eluate was removed.
- A second elution step was performed using 50µl buffer EB (QIAGEN) and incubating for 5min at RT under shaking. The suspension was placed onto a magnetic rack for separation and the obtained eluate was removed. The second elution step was performed in this example to ensure that all bound DNA is recovered.

The following binding buffers were tested according to this protocol of the invention and compared with the reference product (AMPure):

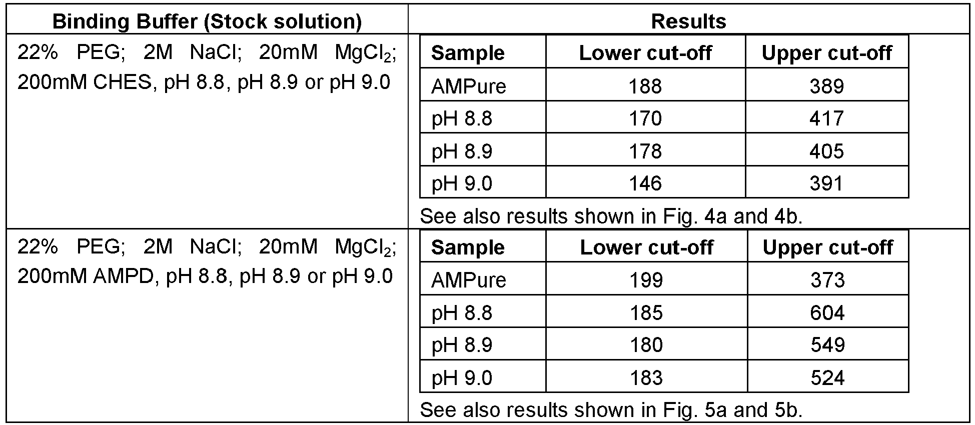

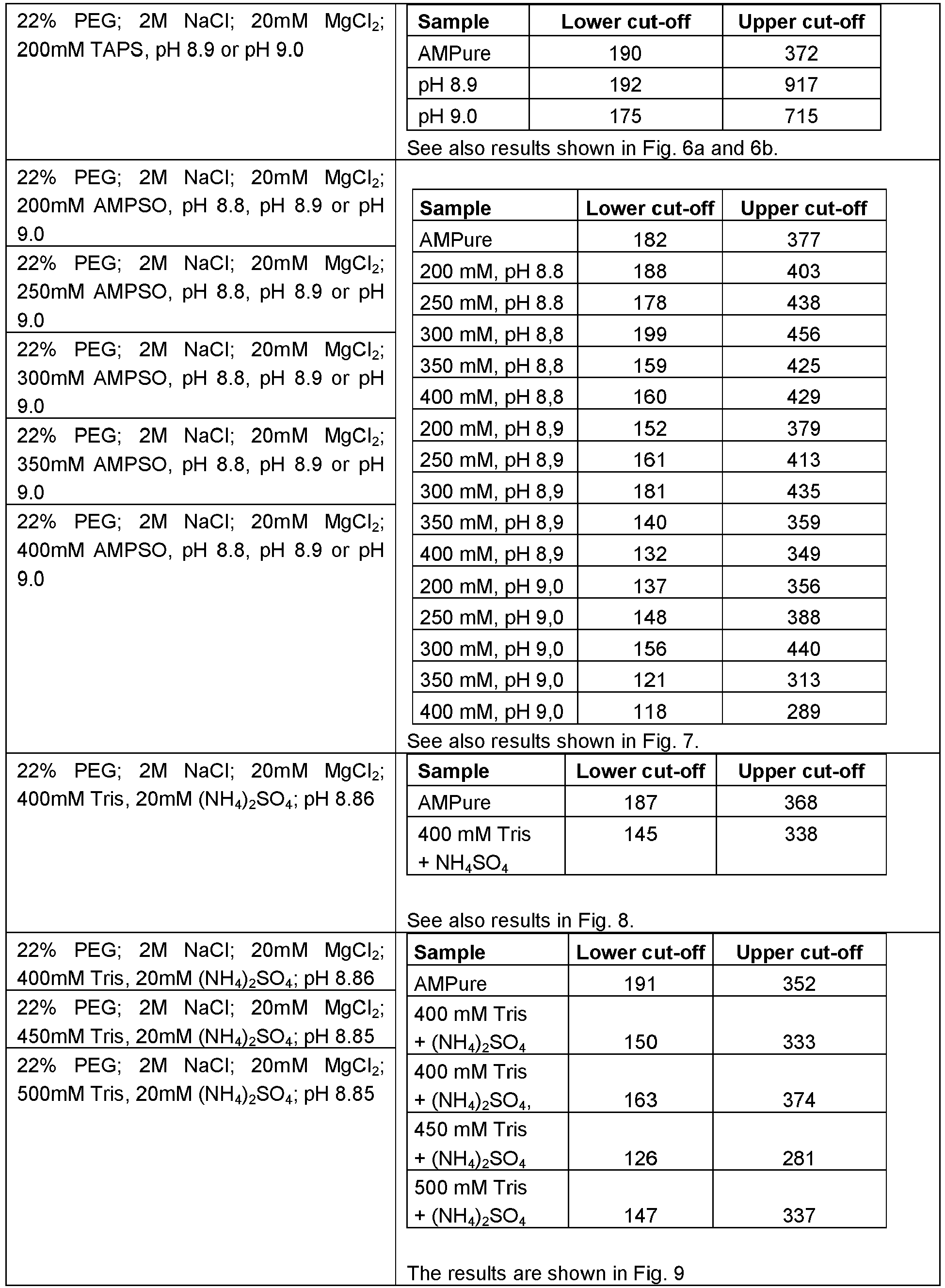

The results demonstrate that various binding buffers can be used in the method according to the invention and provide good results when using a solid phase having a unmodified silicon containing surface for nucleic acid binding. The method of the invention is cost-effective and robust and thereby makes an important contribution to the art.

### 5. Example 5

The example shows cleanup and size selection in a NGS (targeted sequencing) library preparation workflow.

### Experimental design:

Human genomic DNA was used as sample material to generate a DNA sequencing panel which is a set of PCR amplicons as defined by the "Actionable Insights Tumor Panel" (GRTP-101X-12). This gene panel is a set of PCR primers to amplify a set of regions within relevant cancer-related genes to enable targeted sequencing of these DNA regions. The multiplex PCR to amplify the regions of this gene panel was performed according to the "QIAact Gene Panels Handbook" (December 2015; QIAGEN).

The amplified gene panel DNA was then subjected to a cleanup and size selection protocol in order to remove unwanted primers, unspecific amplification products and other impurities.

### Protocol details:

1) To 100 µl of DNA (unpurified PCR product), add 30 µl of undiluted forensic MagAttract Suspension G (QIAGEN) to the sample, bringing the total volume to 130 µl.
2) Add size selection buffer to the sample to 46%* concentration, mix.
3) Incubate at RT with shaking for 20 min.
4) Separate the beads from suspension by a magnetic rack. Transfer supernatant and discard beads.
5) Add 30 µl undiluted forensic MagAttract G beads to the supernatant from step 4.
6) Add size selection buffer to 70%* concentration, mix.
7) Incubate at RT with shaking for 20 min.
8) Separate the beads from suspension on a magnetic rack. Remove and discard supernatant.
9) Add 500 µl of 80% ethanol to the beads, mix.
10) Incubate at RT with shaking for 5 min.
11) Separate the beads from suspension on a magnet stand. Remove and discard supernatant.
12) Repeat steps 9-11 once.
13) Air dry the beads for 20 minutes.
14) Add 50 µl of elution buffer to the beads. Mix by vortexing.
15) Incubate at RT with shaking for 5 min.
16) Separate the beads from suspension on a magnetic rack.
17) Transfer ≈ 50 µl eluate to a new tube.
*percentage of size selection buffer in the binding mixture

### Size selection buffers used:

**Size selection buffers used - general composition:**

| | |
|---|---|
| PEG 6000 | 22% |
| NaCI | 2 M |
| MgCl₂ | 20 mM |
| (NH₄)₂SO₄ | 20 mM |
| buffering agent (pH 9.0 - 9.3) | 250 mM |

Buffering agents used:
1) CAPSO (N-cyclohexyl-3-aminopropanesulfonic acid),
2) AMPD (2-amino-2-methyl-1,3-propanediol)
3) CHES (N-Cyclohexyl-2-aminoethanesulfonic acid)
4) TAPS (N-[Tris(hydroxymethyl)methyl]-3-aminopropanesulfonic acid)

The respective buffers were adjusted to different pH values from 9.0 to 9.3 as further indicated below.

In order to determine the stability of the tested 4 variants of the size selection buffer, the buffers were stored for 6 months at 25°C after they were made up fresh and then used for the DNA cleanup and size selection protocol (Results 2 to 4). As a reference, freshly made buffers using CAPSO and CHES as buffering agents were tested (Results 1).

After cleanup, the DNA was analyzed using an Agilent Bioanalyzer capillary electrophoresis system according to the manufacturer's instructions.

The DNA recovery rate was calculated based on the amount of DNA that was subjected to the cleanup protocol and the amount of gene panel DNA in the targeted size range (as determined using the Agilent BioAnalyzer software).

### Results 1 - Size Selection Buffer with CAPSO / CHES (freshly prepared):

Freshly made buffers using CAPSO and CHES as buffering agents were tested. Using either CAPSO or CHES, the pH of the size selection buffer was adjusted to 9.0, 9.1, 9.2, respectively. After completion of the DNA cleanup and size selection protocol (described above), the DNA was analyzed and quantified using the Agilent BioAnalyzer. The results are shown in Fig. 10a. The figure shows an Agilent BioAnalyzer gel-like image obtained for a ladder (L) and for samples of gene panel DNA. Samples 1-6 were analyzed after cleanup and size selection. Sample 7 is gene panel DNA before cleanup, 1:100 diluted.

| | |
|---|---|
| **Sample 1** | GP CAPSO pH 9.0 |
| **Sample 2** | GP CAPSO pH 9.1 |
| **Sample 3** | GP CAPSO pH 9.2 |
| **Sample 4** | GP CHES pH 9.0 |
| **Sample 5** | GP CHES pH 9.1 |
| **Sample 6** | GP CHES pH 9.2 |
| **Sample 7** | GP before cleanup 1:100 diluted |

As can be seen from a comparison of the purity of the purified gene panel DNA (samples 1-6) with 100-fold diluted unpurified DNA (sample 7), the size selection protocol is well suitable to purify DNA that is derived from a highly multiplexed PCR reaction as it is typically used to amplify a set of DNA regions for Next-Generation Sequencing applications, e.g., in a genetic cancer profiling application.

Fig. 10b shows the DNA recovery rate (%) calculated for gene panel DNA samples 1-6 as described above.

### Results 2 to 4 - Size Selection Buffers with CAPSO, AMPD, CHES or TAPS (6 months of storage):

In order to determine the stability of the tested 4 variants of the size selection buffer (described above, comprising CAPSO, AMPD, CHES or TAPS as buffering agents), the buffers were stored for 6 months at 25°C after they were made up fresh and then used for the DNA cleanup and size selection protocol. The results obtained for the individual buffering agents are provided below and in Figs. 11-14.

### Results 2 - Size Selection Buffer with CAPSO (6 months of storage):

Size selection buffers with CAPSO as buffering agent (pH 9.0 / 9.1 / 9.2) were tested in three replicates each. After completion of the DNA cleanup and size selection protocol (described above), the DNA was analyzed using the Agilent BioAnalyzer. The Agilent BioAnalyzer capillary gel electrophoresis results are shown in Fig. 11a. The figure shows an Agilent BioAnalyzer gel-like image obtained for a ladder (L) and for samples 1-9 of gene panel DNA:

| | | |
|---|---|---|
| **Sample 1** | GP CAPSO 9.0 | 25°C |
| **Sample 2** | GP CAPSO 9.0 | 25°C |
| **Sample 3** | GP CAPSO 9.0 | 25°C |
| **Sample 4** | GP CAPSO 9.1 | 25°C |
| **Sample 5** | GP CAPSO 9.1 | 25°C |
| **Sample 6** | GP CAPSO 9.1 | 25°C |
| **Sample 7** | GP CAPSO 9.2 | 25°C |
| **Sample 8** | GP CAPSO 9.2 | 25°C |
| **Sample 9** | GP CAPSO 9.2 | 25°C |

Fig. 11b shows the DNA recovery rate (%) calculated for gene panel DNA samples 1-9.

### Results 3 - Size Selection Buffer with AMPD (6 months of storage):

Size selection buffers with AMPD as buffering agent (pH 9.0 / 9.1) were tested in three replicates each. After completion of the DNA cleanup and size selection protocol (described above), the DNA was analyzed using the Agilent BioAnalyzer. The Agilent BioAnalyzer capillary gel electrophoresis results are shown in Fig. 12a. The figure shows an Agilent BioAnalyzer gel-like image obtained for a ladder (L) and for samples 1-6 of gene panel DNA:

| | | |
|---|---|---|
| **Sample 1** | GP AMPD 9.0 | 25°C |
| **Sample 2** | GP AMPD 9.0 | 25°C |
| **Sample 3** | GP AMPD 9.0 | 25°C |
| **Sample 4** | GP AMPD 9.1 | 25°C |
| **Sample 5** | GP AMPD 9.1 | 25°C |
| **Sample 6** | GP AMPD 9.1 | 25°C |

Fig. 12b shows the DNA recovery rate (%) calculated for gene panel DNA samples 1-6.

### Results 4 - Size Selection Buffer with CHES (6 months of storage):

Size selection buffer with CHES as buffering agent (pH 9.0 / 9.1 / 9.2) was tested in three replicates each. After completion of the DNA cleanup and size selection protocol (described above), the DNA was analyzed using the Agilent BioAnalyzer. The Agilent BioAnalyzer capillary gel electrophoresis results are shown in Fig. 13a. The figure shows an Agilent BioAnalyzer gel-like image obtained for a ladder (L) and for samples 1-9 of gene panel DNA:

| | | |
|---|---|---|
| **Sample 1** | GP CHES 9.0 | 25°C |
| **Sample 2** | GP CHES 9.0 | 25°C |
| **Sample 3** | GP CHES 9.0 | 25°C |
| **Sample 4** | GP CHES 9.1 | 25°C |
| **Sample 5** | GP CHES 9.1 | 25°C |
| **Sample 6** | GP CHES 9.1 | 25°C |
| **Sample 7** | GP CHES 9.2 | 25°C |
| **Sample 8** | GP CHES 9.2 | 25°C |
| **Sample 9** | GP CHES 9.2 | 25°C |

Fig. 13b shows the DNA recovery rate (%) calculated for gene panel DNA samples 1-9.

### Results 5 - Size Selection Buffer with TAPS (6 months of storage):

Size selection buffer with TAPS as buffering agent (pH 9.0 / 9.3) was tested in three replicates each. After completion of the DNA cleanup and size selection protocol (described above), the DNA was analyzed using the Agilent BioAnalyzer. The Agilent BioAnalyzer capillary gel electrophoresis results are shown in Fig. 14a. The figure shows an Agilent BioAnalyzer gel-like image obtained for a ladder (L) and for samples 1-6 of gene panel DNA:

| | | |
|---|---|---|
| **Sample 1** | GP TAPS 9.0 | 25°C |
| **Sample 2** | GP TAPS 9.0 | 25°C |
| **Sample 3** | GP TAPS 9.0 | 25°C |
| **Sample 4** | GP TAPS 9.3 | 25°C |
| **Sample 5** | GP TAPS 9.3 | 25°C |
| **Sample 6** | GP TAPS 9.3 | 25°C |

Fig. 14b shows the DNA recovery rate (%) calculated for gene panel DNA samples 1-6.

### Conclusions:

All four tested buffering agents are suitable for a DNA size selection buffer to bind DNA to a silica surface in a size-dependent manner.

As shown in the "Results 1" section (comparison of the purity of the purified gene panel DNA against 100-fold diluted unpurified DNA), the size selection protocol is well suitable to purify DNA that is derived from a highly multiplexed PCR reaction as it is typically used to amplify a set of DNA regions for Next-Generation Sequencing applications, e.g., in a genetic cancer profiling application.

The DNA recovery is improved at alkaline pH. A pH of 9.2 or 9.3 leads to even higher DNA recovery rates than a pH of 9.0.

The tested buffer variants were found to work well. The size selection buffer variants with
- CAPSO, pH 9.2
- AMPD, pH 9.1
- CHES, pH 9.2
- TAPS, pH 9.3
show the highest DNA recovery rates after 6 months storage at 25°C (Results 2 to 4). The recovery rates are also comparable to the DNA recovery rate obtained with freshly made size selection buffer containing CAPSO or CHES (Results 1).

## Claims

1. A poly(alkylene oxide) polymer based size selective DNA isolation method for isolating DNA molecules having a size above a certain cut-off value from a DNA containing sample, comprising
(a) preparing a binding mixture comprising the DNA containing sample, at least one poly(alkylene oxide) polymer which is a polyethylene glycol, at least one alkali metal salt and at least one divalent cation, wherein the binding mixture comprises the divalent cation in a concentration of 3mM to 75mM, and wherein said binding mixture has a pH that lies in the range of 8.5 to 9.5 and binding precipitated DNA molecules to a solid phase having an unmodified silicon containing surface, thereby providing a solid phase having bound thereto DNA molecules having a size above the cut-off value, wherein under the used binding conditions DNA molecules having a size which is less than the cut-off value predominantly do not bind to the solid phase;
(b) separating the bound DNA molecules from the remaining sample;
- optionally washing the bound DNA molecules; and
- optionally eluting the bound DNA molecules from the solid phase.

2. The method according to claim 1 for isolating target DNA molecules having a size within a certain size range that is determined by an upper cut-off value and a lower cut-off value from a DNA containing sample, comprising
(a) preparing a first binding mixture comprising the DNA containing sample, at least one poly(alkylene oxide) polymer which is a polyethylene glycol, at least one alkali metal salt and at least one divalent cation, wherein the binding mixture comprises the divalent cation in a concentration of 3mM to 75mM, and wherein said first binding mixture has a pH that lies in the range of 8.5 to 9.5 and binding precipitated DNA molecules to a solid phase having an unmodified silicon containing surface, thereby providing a solid phase having bound thereto DNA molecules having a size above the upper cut-off value, wherein under the used binding conditions target DNA molecules having a size which is less than the upper cut-off value predominantly do not bind to the solid phase;
(b) separating the bound DNA molecules from the remaining sample which comprises the target DNA molecules;
(c) preparing a second binding mixture comprising the remaining sample, wherein said second binding mixture has a pH that lies in the range of 8.5 to 9.5 and wherein the concentration of the poly(alkylene oxide) polymer which is a polyethylene glycol in the second binding mixture is increased compared to the first binding mixture and binding precipitated target DNA molecules to a solid phase having an unmodified silicon containing surface, thereby providing a solid phase having bound thereto target DNA molecules having a size above the lower cut-off value, wherein under the used binding conditions DNA molecules having a size which is less than the lower cut-off value predominantly do not bind to the solid phase;
(d) separating the bound target DNA molecules from the remaining sample;
- optionally washing the bound target DNA molecules; and
- optionally eluting the bound target DNA molecules from the solid phase.

3. The method according to claim 1 or 2, wherein in step (a) a binding buffer is added to the DNA containing sample to prepare the binding mixture, wherein the binding buffer comprises the at least one poly(alkylene oxide) polymer which is a polyethylene glycol, the at least one divalent cation and at least one buffering agent, wherein said binding buffer has a pH that lies in a range of 8.5 to 9.5, and wherein optionally the binding buffer additionally comprises the at least one alkali metal salt.

4. The method according to claim 2 or 3, wherein in step (c) a binding buffer is added to the remaining sample to prepare the second binding mixture, wherein the binding buffer comprises the at least one poly(alkylene oxide) polymer which is a polyethylene glycol, at least one divalent cation and at least one buffering agent and wherein said binding buffer has a pH that lies in a range of 8.5 to 9.5 and wherein optionally, the binding buffer additionally comprises at least one alkali metal salt.

5. The method according to claim 4, wherein the same binding buffer is used as in step (a).

6. The method according to any one of claims 1 to 5, wherein the alkali metal salt has one or more of the following characteristics:
a) the alkali metal salt is an alkali metal halide;
b) the alkali metal salt is sodium chloride; and/or
c) the alkali metal salt is present in the binding mixture in a concentration selected from the ranges 250mM to 2M, 350mM to 1.75M, 500mM to 1.5M, 600mM to 1.3M and 650mM to 1.25M.

7. The method according to any one of claims 1 to 6, wherein the divalent cation has one or more of the following characteristics:
a) the divalent cation is selected from Mg²⁺, Fe²⁺, Ca²⁺, Mn²⁺, Zn²⁺ and Ni²⁺ and preferably is Mg²⁺;
b) the divalent cation is present in the binding mixture in form of a dissolved salt, wherein said salt optionally is a halide;
c) the divalent cation is present in the binding mixture in form of a dissolved salt which is magnesium chloride;
d) the divalent cation is present in the binding mixture in a concentration selected from the ranges 4mM to 50mM, 5mM to 40mM, 5.5mM to 35mM, 6mM to 30mm, 6.5mM to 25mM, 7mM to 20mM and 7.5mM to 15mM; and/or
e) the divalent cation is present in the binding mixture in form of a dissolved salt and the binding mixture additionally contains sodium chloride.

8. The method according to any one of claims 1 to 7, having one or more of the following characteristics:
a) the polyethylene glycol has a molecular weight that is selected from the ranges 2000 to 40000, 2500 to 30000, 3000 to 25000, 3500 to 20000, 4000 to 16000, 4500 to 12000 and 5000 to 10000;
b) the cut-off value is adjusted by the concentration of the polyethylene glycol in the binding mixture; and/or
c) the binding mixture comprises the polyethylene glycol in a concentration in a range selected from 6% to 20%, 7% to 17%, 7.5% to 15%, 8% to 13% and 8.5% to 12.5%.

9. The method according to any one of claims 1 to 8, wherein the binding mixture has a pH value that lies in a range selected from 8.6 to 9.2 and/or wherein the binding mixture comprises at least one buffering agent, preferably in a concentration selected from 25mM to 600mM, 50mM to 500mM, 60mM to 450, 70mM to 400mM, 80mM to 350mM, 90mM to 300mM and 100mM to 275mM.

10. The method according to any one of claims 3 to 9, wherein the binding conditions are exclusively established by the binding buffer that is contacted with the DNA containing sample and/or the remaining sample.

11. The method according to any one of claims 3 to 5 or the method according to any one of claims 6 to 10 when depending on any one of claims 3 to 5, wherein the binding buffer has one or more of the following characteristics:
a) it has a pH value that lies in a range selected from 8.6 to 9.2;
b) it comprises a buffering agent in a concentration selected from 25mM to 1M, 50mM to 750mM, 75mM to 700mM, 100mM to 650mM, 125mM to 600mM, 150mM to 550mM, 175mM to 525mM and 200mM to 500mM;
c) it comprises the alkali metal salt in a concentration selected from 0.5M to 4M, 0.7M to 3.5M, 1M to 3M, 1.2M to 2.75M and 1.3M to 2.5M;
d) it comprises the polyethylene glycol in a concentration selected from 7% to 45%, 10% to 40%, 12.5% to 35%, 15% to 30%, 17.5% to 27.5% and 20% to 25%.

12. The method according to any one of claims 3 to 5 or the method according to any one of claims 6 to 10 when depending on any one of claims 3 to 5, wherein the binding buffer has the following features:
i) it comprises Mg²⁺ as divalent cation, preferably as MgCl₂;
ii) it comprises polyethylene glycol in a concentration selected from 12.5% to 35%, 15% to 30%, 17.5% to 27.5% and 20% to 25%;
iii) it comprises the alkali metal salt, preferably sodium chloride, in a concentration that lies in the range of 0.5M to 2.75M;
iv) it comprises the at least one buffering agent in a concentration that lies in a range of 100 to 550mM;
v) it has a pH that lies in the range of 8.5 to 9.5 or 8.6 to 9.2.

13. The method according to any one of claims 1 and 3 to 12, wherein the method is performed as follows:
(a) preparing a binding mixture comprising the DNA containing sample, a polyethylene glycol in a concentration that lies in the range of 7.5% to 15%, preferably 8% to 13%, and MgCl₂ in a concentration that lies in the range of 7mM to 40mM, preferably 8mM to 20mM, wherein said binding mixture has a pH that lies in the range of 8.5 to 9.25 and binding precipitated DNA molecules to a solid phase having an unmodified silicon containing surface, thereby providing a solid phase having bound thereto DNA molecules having a size above the cut-off value, wherein under the used binding conditions DNA molecules having a size which is less than the cut-off value predominantly do not bind to the solid phase;
(b) separating the bound DNA molecules from the remaining sample;
- optionally washing the bound DNA molecules; and
- optionally eluting the bound DNA molecules from the solid phase.

14. The method according to any one of claims 2 to 13, wherein the method is performed as follows:
(a) preparing a first binding mixture comprising the DNA containing sample, polyethylene glycol in a concentration that lies in the range of 8.5% to 9.5% and MgCl₂ in a concentration that lies in the range of 7mM to 40mM, preferably 8mM to 20mM, wherein said binding mixture has a pH that lies in the range of 8.5 to 9.25 and binding precipitated DNA molecules to a solid phase having an unmodified silicon containing surface, thereby providing a solid phase having bound thereto DNA molecules having a size above the upper cut-off value, wherein under the used binding conditions target DNA molecules having a size which is less than the upper cut-off value predominantly do not bind to the solid phase;
(b) separating the bound DNA molecules from the remaining sample which comprises the target DNA molecules;
(c) preparing a second binding mixture comprising the remaining sample, wherein said second binding mixture has a pH that lies in the range of 8.5 to 9.25 and wherein the concentration of polyethylene glycol in the second binding mixture is increased compared to the first binding mixture and lies in a range of 10% to 12% and binding precipitated target DNA molecules to a solid phase having an unmodified silicon containing surface, thereby providing a solid phase having bound thereto target DNA molecules, wherein under the used binding conditions DNA molecules having a size which is less than the lower cut-off value predominantly do not bind to the solid phase;
(d) separating the bound target DNA molecules from the remaining sample;
- optionally washing the bound target DNA molecules; and
- optionally eluting the bound target DNA molecules from the solid phase.

15. The method according to any one of claims 1 to 14, for isolating adapter ligated DNA molecules as target DNA molecules from a DNA containing sample which is an adapter ligation sample and for removing adapter monomers and adapter-adapter ligation products, wherein adapter ligated DNA molecules are separated from unligated adapter monomers and adapter-adapter ligation products based on the larger size of the adapter ligated DNA molecules.

16. The method according to any one of claims 1 to 15, wherein the solid phase has one or more of the following characteristics:
a) the solid phase provides a siliceous surface;
b) the solid phase provides a silica surface;
c) the solid phase is provided in form of a membrane, filter, sheet or particles;
d) the solid phase is column based or is provided in form of magnetic particles.

17. A kit for the size selective isolation of target DNA molecules having a size above a desired cut-off value from a DNA containing sample, wherein the kit is suitable for the method according to claim 1, the kit comprising
(a) a binding buffer comprising at least one poly(alkylene oxide) polymer which is a polyethylene glycol, at least one alkali metal salt, at least one divalent cation and at least one buffering agent wherein the binding buffer has a pH value that lies in the range of 8.5 to 9.5;
(b) a solid phase having an unmodified silicon containing surface;
(c) optionally at least one washing solution; and
(d) optionally at least one an elution solution.

18. The kit according to claim 17, wherein the binding buffer has one or more of the characteristics as specified in claim 11 and/or wherein the solid phase has one or more of the characteristics defined in claim 16.

## Patentansprüche

1. Ein Poly(alkylenoxid)-Polymer basiertes größenselektives DNA-Isolierungsverfahren zur Isolierung von DNA-Molekülen mit einer Größe oberhalb eines bestimmten Grenzwertes aus einer DNA enthaltenden Probe, umfassend
(a) Vorbereiten einer Bindungsmischung umfassend die DNA enthaltende Probe, mindestens ein Poly(alkylenoxid)-Polymer, das ein Polyethylenglykol ist, mindestens ein Alkalimetallsalz und mindestens ein zweiwertiges Kation, wobei das Bindungsgemisch das zweiwertige Kation in einer Konzentration von 3mM bis 75mM enthält und wobei das Bindungsgemisch einen pH-Wert aufweist, der im Bereich von 8,5 bis 9,5 liegt, und Binden ausgefällter DNA-Moleküle an eine feste Phase mit einer unmodifizierten, siliziumhaltigen Oberfläche, wodurch eine feste Phase bereitgestellt wird, an die DNA-Moleküle mit einer Größe oberhalb des Grenzwertes gebunden sind, wobei unter den verwendeten Bindungsbedingungen DNA-Moleküle mit einer Größe, die kleiner ist als der Grenzwert, überwiegend nicht an die feste Phase binden;
(b) Abtrennen der gebundenen DNA-Moleküle von der restlichen Probe;
- optionales Waschen der gebundenen DNA-Moleküle; und
- optionales Eluieren der gebundenen DNA-Moleküle von der festen Phase.

2. Das Verfahren nach Anspruch 1 zur Isolierung von Ziel-DNA-Molekülen mit einer Größe innerhalb eines bestimmten Größenbereichs, der durch einen oberen Grenzwert und einen unteren Grenzwert bestimmt wird, aus einer DNA enthaltenden Probe, umfassend
(a) Vorbereiten einer ersten Bindungsmischung umfassend die DNA enthaltende Probe, mindestens ein Poly(alkylenoxid)-Polymer, das ein Polyethylenglykol ist, mindestens ein Alkalimetallsalz und mindestens ein zweiwertiges Kation, wobei das Bindungsgemisch das zweiwertige Kation in einer Konzentration von 3mM bis 75mM enthält und wobei dieses erste Bindungsgemisch einen pH-Wert aufweist, der im Bereich von 8,5 bis 9,5 liegt, und Binden ausgefällter DNA-Moleküle an eine feste Phase mit einer unmodifizierten, siliziumhaltigen Oberfläche, wodurch eine feste Phase bereitgestellt wird, an die DNA-Moleküle mit einer Größe oberhalb des oberen Grenzwertes gebunden sind, wobei unter den verwendeten Bindungsbedingungen Ziel-DNA-Moleküle mit einer Größe, die kleiner ist als der obere Grenzwert, überwiegend nicht an die feste Phase binden;
(b) Abtrennen der gebundenen DNA-Moleküle von der restlichen Probe, die die Ziel-DNA-Moleküle enthält;
(c) Vorbereiten einer zweiten Bindungsmischung umfassend die verbleibende Probe, wobei die zweite Bindungsmischung einen pH-Wert aufweist, der im Bereich von 8,5 bis 9,5 liegt, und wobei die Konzentration des Poly(alkylenoxid)-Polymers, das ein Polyethylenglykol ist, in der zweiten Bindungsmischung im Vergleich zur ersten Bindungsmischung erhöht ist, und Binden ausgefällter Ziel-DNA-Moleküle an eine feste Phase mit einer unmodifizierten, siliziumhaltigen Oberfläche, wodurch eine feste Phase bereitgestellt wird, an die Ziel-DNA-Moleküle mit einer Größe oberhalb des unteren Grenzwertes gebunden sind, wobei unter den verwendeten Bindungsbedingungen DNA-Moleküle mit einer Größe, die kleiner ist als der untere Grenzwert, überwiegend nicht an die feste Phase binden;
(d) Abtrennen der gebundenen Ziel-DNA-Moleküle von der restlichen Probe;
- optionales Waschen der gebundenen Ziel-DNA-Moleküle; und
- optionales Eluieren der gebundenen Ziel-DNA-Moleküle von der festen Phase.

3. Das Verfahren nach Anspruch 1 oder 2, wobei in Schritt (a) ein Bindungspuffer zu der DNA-enthaltenden Probe gegeben wird, um die Bindungsmischung vorzubereiten, wobei der Bindungspuffer das mindestens eine Poly(alkylenoxid)-Polymer, das ein Polyethylenglykol ist, das mindestens eine zweiwertige Kation und mindestens ein Puffermittel umfasst, wobei der Bindungspuffer einen pH-Wert aufweist, der im Bereich von 8,5 bis 9,5 liegt, und wobei der Bindungspuffer optional zusätzlich das mindestens eine Alkalimetallsalz enthält.

4. Das Verfahren nach Anspruch 2 oder 3, wobei in Schritt (c) ein Bindungspuffer zu der verbleibenden Probe gegeben wird, um die zweite Bindungsmischung vorzubereiten, wobei der Bindungspuffer das mindestens eine Poly(alkylenoxid)-Polymer, das ein Polyethylenglykol ist, mindestens ein zweiwertiges Kation und mindestens ein Puffermittel umfasst und wobei der Bindungspuffer einen pH-Wert aufweist, der in einem Bereich von 8,5 bis 9,5 liegt, und wobei der Bindungspuffer optional zusätzlich mindestens ein Alkalimetallsalz enthält.

5. Das Verfahren nach Anspruch 4, wobei der gleiche Bindungspuffer wie in Schritt (a) verwendet wird.

6. Das Verfahren nach einem der Ansprüche 1 bis 5, wobei das Alkalimetallsalz eine oder mehrere der folgenden Eigenschaften aufweist:
a) das Alkalimetallsalz ist ein Alkalimetallhalogenid;
b) das Alkalimetallsalz ist Natriumchlorid; und/oder
c) das Alkalimetallsalz ist in der Bindungsmischung in einer Konzentration vorhanden, die aus den Bereichen 250mM bis 2M, 350mM bis 1,75M, 500mM bis 1,5M, 600mM bis 1,3M und 650mM bis 1,25M ausgewählt ist.

7. Das Verfahren nach einem der Ansprüche 1 bis 6, wobei das zweiwertige Kation eine oder mehrere der folgenden Eigenschaften aufweist:
a) das zweiwertige Kation ist ausgewählt aus Mg²⁺, Fe²⁺, Ca²⁺, Mn²⁺, Zn²⁺ und Ni²⁺ und ist vorzugsweise Mg²⁺;
b) das zweiwertige Kation ist in der Bindungsmischung in Form eines gelösten Salzes vorhanden, wobei das Salz optional ein Halogenid ist;
c) das zweiwertige Kation liegt in der Bindungsmischung in Form eines gelösten Salzes vor, welches Magnesiumchlorid ist;
d) das zweiwertige Kation in der Bindungsmischung ist in einer Konzentration vorhanden, die aus den Bereichen 4mM bis 50mM, 5mM bis 40mM, 5,5mM bis 35mM, 6mM bis 30mM, 6,5mM bis 25mM, 7mM bis 20mM und 7,5mM bis 15mM ausgewählt ist; und/oder
e) das zweiwertige Kation liegt in der Bindungsmischung in Form eines gelösten Salzes vor und die Bindungsmischung enthält zusätzlich Natriumchlorid.

8. Das Verfahren nach einem der Ansprüche 1 bis 7, mit einer oder mehreren der folgenden Eigenschaften:
a) Das Polyethylenglykol hat ein Molekulargewicht, das ausgewählt ist aus den Bereichen 2000 bis 40000, 2500 bis 30000, 3000 bis 25000, 3500 bis 20000, 4000 bis 16000, 4500 bis 12000 und 5000 bis 10000;
b) der Grenzwert wird durch die Konzentration des Polyethylenglykols in der Bindungsmischung eingestellt; und/oder
c) die Bindungsmischung enthält das Polyethylenglykol in einer Konzentration in einem Bereich, der ausgewählt wird aus 6% bis 20%, 7% bis 17%, 7,5% bis 15%, 8% bis 13% und 8,5% bis 12,5%.

9. Das Verfahren nach einem der Ansprüche 1 bis 8, wobei die Bindungsmischung einen pH-Wert aufweist, der in einem Bereich zwischen 8,6 und 9,2 liegt, und/oder wobei die Bindungsmischung mindestens eine Puffersubstanz enthält, vorzugsweise in einer Konzentration, die ausgewählt ist aus 25mM bis 600mM, 50mM bis 500mM, 60mM bis 450, 70mM bis 400mM, 80mM bis 350mM, 90mM bis 300mM und 100mM bis 275mM.

10. Das Verfahren nach einem der Ansprüche 3 bis 9, wobei die Bindungsbedingungen ausschließlich durch den Bindungspuffer hergestellt werden, der mit der DNA-enthaltenden Probe und/oder der restlichen Probe in Kontakt gebracht wird.

11. Das Verfahren nach einem der Ansprüche 3 bis 5 oder das Verfahren nach einem der Ansprüche 6 bis 10 wenn abhängig von einem der Ansprüche 3 bis 5, wobei der Bindungspuffer eine oder mehrere der folgenden Eigenschaften aufweist:
a) er weist einen pH-Wert auf, der in einem Bereich zwischen 8,6 und 9,2 liegt;
b) er enthält eine Puffersubstanz in einer Konzentration, die ausgewählt ist aus 25mM bis 1M, 50mM bis 750mM, 75mM bis 700mM, 100mM bis 650mM, 125mM bis 600mM, 150mM bis 550mM, 175mM bis 525mM und 200mM bis 500mM;
c) er enthält das Alkalimetallsalz in einer Konzentration, die ausgewählt ist aus 0,5M bis 4M, 0,7M bis 3,5M, 1M bis 3M, 1,2M bis 2,75M und 1,3M bis 2,5M;
d) er enthält das Polyethylenglykol in einer Konzentration, die ausgewählt ist aus 7% bis 45%, 10% bis 40%, 12,5% bis 35%, 15% bis 30%, 17,5% bis 27,5% und 20% bis 25%.

12. Das Verfahren nach einem der Ansprüche 3 bis 5 oder das Verfahren nach einem der Ansprüche 6 bis 10 wenn abhängig von einem der Ansprüche 3 bis 5, wobei der Bindungspuffer die folgenden Merkmale aufweist:
i) er enthält Mg²⁺ als zweiwertiges Kation, vorzugsweise als MgCl₂;
ii) er enthält Polyethylenglykol in einer Konzentration, die ausgewählt ist aus 12,5% bis 35%, 15% bis 30%, 17,5% bis 27,5% und 20% bis 25%;
iii) er enthält das Alkalimetallsalz, vorzugsweise Natriumchlorid, in einer Konzentration, die in einem Bereich von 0,5M bis 2,75M liegt;
iv) er enthält das mindestens eine Puffermittel in einer Konzentration, die in einem Bereich von 100 bis 550mM liegt;
v) er weist einen pH-Wert auf, der in einem Bereich von 8,5 bis 9,5 oder 8,6 bis 9,2 liegt.

13. Das Verfahren nach einem der Ansprüche 1 und 3 bis 12, wobei das Verfahren wie folgt durchgeführt wird:
(a) Vorbereiten einer Bindungsmischung umfassend die DNA-enthaltende Probe, ein Polyethylenglykol in einer Konzentration, die im Bereich von 7,5 % bis 15 % liegt, vorzugsweise 8% bis 13%, und MgCl₂ in einer Konzentration, die im Bereich von 7mM bis 40mM liegt, vorzugsweise 8mM bis 20mM, wobei die Bindungsmischung einen pH-Wert aufweist, der im Bereich von 8,5 bis 9,25 liegt und Binden ausgefällter DNA-Moleküle an eine feste Phase mit einer unmodifizierten, siliziumhaltigen Oberfläche, wodurch eine feste Phase bereitgestellt wird, an die DNA-Moleküle mit einer Größe oberhalb des Grenzwertes gebunden sind, wobei unter den verwendeten Bindungsbedingungen DNA-Moleküle mit einer Größe, die kleiner ist als der Grenzwert, überwiegend nicht an die feste Phase binden;
(b) Abtrennen der gebundenen DNA-Moleküle von der restlichen Probe;
- optionales Waschen der gebundenen DNA-Moleküle; und
- optionales Eluieren der gebundenen DNA-Moleküle von der festen Phase.

14. Das Verfahren nach einem der Ansprüche 2 bis 13, wobei das Verfahren wie folgt durchgeführt wird:
(a) Vorbereiten einer ersten Bindungsmischung umfassend die DNA-enthaltende Probe, Polyethylenglykol in einer Konzentration, die im Bereich von 8,5 % bis 9,5 % liegt, und MgCl₂ in einer Konzentration, die im Bereich von 7mM bis 40mM liegt, vorzugsweise 8mM bis 20mM, wobei die Bindungsmischung einen pH-Wert aufweist, der im Bereich von 8,5 bis 9,25 liegt, und Binden ausgefällter DNA-Moleküle an eine feste Phase mit einer unmodifizierten, siliziumhaltigen Oberfläche, wodurch eine feste Phase bereitgestellt wird, an die DNA-Moleküle mit einer Größe oberhalb des oberen Grenzwertes gebunden sind, wobei unter den verwendeten Bindungsbedingungen Ziel-DNA-Moleküle mit einer Größe, die kleiner ist als der obere Grenzwert, überwiegend nicht an die feste Phase binden;
(b) Abtrennen der gebundenen DNA-Moleküle von der restlichen Probe, die die Ziel-DNA-Moleküle enthält;
(c) Vorbereiten einer zweiten Bindungsmischung umfassend die verbleibende Probe, wobei diese zweite Bindungsmischung einen pH-Wert aufweist, der im Bereich von 8,5 bis 9,25 liegt, und wobei die Konzentration von Polyethylenglykol in der zweiten Bindungsmischung im Vergleich zur ersten Bindungsmischung erhöht ist und in einem Bereich von 10 % bis 12 % liegt, und Binden ausgefällter Ziel-DNA-Moleküle an eine feste Phase mit einer unmodifizierten, siliziumhaltigen Oberfläche, wodurch eine feste Phase bereitgestellt wird, an die Ziel-DNA-Moleküle gebunden sind, wobei unter den verwendeten Bindungsbedingungen DNA-Moleküle mit einer Größe, die kleiner ist als der untere Grenzwert, überwiegend nicht an die feste Phase binden;
(d) Abtrennen der gebundenen Ziel-DNA-Moleküle von der restlichen Probe;
- optionales Waschen der gebundenen Ziel-DNA-Moleküle; und
- optionales Eluieren der gebundenen Ziel-DNA-Moleküle von der festen Phase.

15. Das Verfahren nach einem der Ansprüche 1 bis 14, zur Isolierung von Adapter-ligierten DNA-Molekülen als Ziel-DNA-Moleküle aus einer DNA-enthaltenden Probe, die eine Adapter-Ligationsprobe ist, und zur Entfernung von Adapter-Monomeren und Adapter-Adapter-Ligationsprodukten, wobei Adapter-ligierte DNA-Moleküle von nicht ligierten Adaptermonomeren und Adapter-Adapter-Ligationsprodukten aufgrund der größeren Größe der Adapter-ligierten DNA-Moleküle getrennt werden.

16. Das Verfahren nach einem der Ansprüche 1 bis 15, wobei die feste Phase eine oder mehrere der folgenden Eigenschaften aufweist:
a) die feste Phase stellt eine silikatische Oberfläche bereit;
b) die feste Phase stellt eine Siliziumdioxidoberfläche bereit;
c) die feste Phase wird in Form einer Membran, eines Filters, einer Folie oder von Partikeln bereitgestellt;
d) die feste Phase ist auf Säulenbasis oder wird in Form von magnetischen Partikeln bereitgestellt.

17. Kit für die größenselektive Isolierung von Ziel-DNA-Molekülen mit einer Größe oberhalb eines gewünschten Grenzwertes aus einer DNA-enthaltenden Probe, wobei das Kit für das Verfahren nach Anspruch 1 geeignet ist, das Kit umfasst
(a) einen Bindungspuffer umfassend mindestens ein Poly(alkylenoxid)-Polymer, das ein Polyethylenglykol ist, mindestens ein Alkalimetallsalz, mindestens ein zweiwertiges Kation und mindestens ein Puffermittel, wobei der Bindungspuffer einen pH-Wert hat, der im Bereich von 8,5 bis 9,5 liegt;
(b) eine feste Phase mit einer unmodifizierten, siliziumhaltigen Oberfläche;
(c) optional mindestens eine Waschlösung; und
(d) optional mindestens eine Elutionslösung.

18. Das Kit nach Anspruch 17, wobei der Bindungspuffer eine oder mehrere der in Anspruch 11 angegebenen Eigenschaften aufweist und/oder wobei die feste Phase eine oder mehrere der in Anspruch 16 definierten Eigenschaften aufweist.

## Revendications

1. Procédé d'isolement d'ADN sélectif en termes de taille sur la base d'un polymère de poly(oxyde d'alkylène) pour isoler des molécules d'ADN qui présentent une taille au-delà d'une certaine valeur de coupure vis-à-vis d'un échantillon contenant de l'ADN, comprenant :
(a) la préparation d'un mélange de liaison qui comprend l'échantillon contenant de l'ADN, au moins un polymère de poly(oxyde d'alkylène) qui est un polyéthylène glycol, au moins un sel de métaux alcalins ou de métal alcalin et au moins un cation divalent, dans lequel le mélange de liaison comprend le cation divalent selon une concentration de 3 mM à 75 mM, et dans lequel ledit mélange de liaison présente un pH qui se situe à l'intérieur de la plage qui va de 8,5 à 9,5, et la liaison de molécules d'ADN précipitées selon une phase solide qui comporte une surface contenant du silicium non modifiée, d'où ainsi l'obtention d'une phase solide à laquelle sont liées les molécules d'ADN qui présentent une taille au-delà de la valeur de coupure, dans lequel, sous les conditions de liaison utilisées, les molécules d'ADN qui présentent une taille qui est inférieure à la valeur de coupure ne sont pas liées de façon prédominante selon la phase solide ; et
(b) la séparation des molécules d'ADN liées vis-à-vis du reste de l'échantillon ;
- en option, le lavage des molécules d'ADN liées ; et
- en option, l'élution des molécules d'ADN liées par rapport à la phase solide.

2. Procédé selon la revendication 1 pour isoler des molécules d'ADN cibles qui présentent une taille à l'intérieur d'une certaine plage de tailles qui est déterminée par une valeur de coupure supérieure et par une valeur de coupure inférieure vis-à-vis d'un échantillon contenant de l'ADN, comprenant :
(a) la préparation d'un premier mélange de liaison qui comprend l'échantillon contenant de l'ADN, au moins un polymère de poly(oxyde d'alkylène) qui est un polyéthylène glycol, au moins un sel de métaux alcalins ou de métal alcalin et au moins un cation divalent, dans lequel le premier mélange de liaison comprend le cation divalent selon une concentration de 3 mM à 75 mM, et dans lequel ledit premier mélange de liaison présente un pH qui se situe à l'intérieur de la plage qui va de 8,5 à 9,5, et la liaison de molécules d'ADN précipitées selon une phase solide qui comporte une surface contenant du silicium non modifiée, d'où ainsi l'obtention d'une phase solide à laquelle sont liées les molécules d'ADN qui présentent une taille au-delà de la valeur de coupure supérieure, dans lequel, sous les conditions de liaison utilisées, les molécules d'ADN cibles qui présentent une taille qui est inférieure à la valeur de coupure supérieure ne sont pas liées de façon prédominante selon la phase solide ;
(b) la séparation des molécules d'ADN liées vis-à-vis du reste de l'échantillon qui comprend les molécules d'ADN cibles ;
(c) la préparation d'un second mélange de liaison qui comprend le reste de l'échantillon, dans lequel ledit second mélange de liaison présente un pH qui se situe à l'intérieur de la plage qui va de 8,5 à 9,5 et dans lequel la concentration du polymère de poly(oxyde d'alkylène) qui est un polyéthylène glycol dans le second mélange de liaison est augmentée par comparaison avec le premier mélange de liaison, et la liaison de molécules d'ADN cibles précipitées selon une phase solide qui comporte une surface contenant du silicium non modifiée, d'où ainsi l'obtention d'une phase solide à laquelle sont liées les molécules d'ADN cibles qui présentent une taille au-delà de la valeur de coupure inférieure, dans lequel, sous les conditions de liaison utilisées, les molécules d'ADN qui présentent une taille qui est inférieure à la valeur de coupure inférieure ne sont pas liées de façon prédominante selon la phase solide ; et
(d) la séparation des molécules d'ADN cibles liées vis-à-vis du reste de l'échantillon;
- en option, le lavage des molécules d'ADN cibles liées ; et
- en option, l'élution des molécules d'ADN cibles liées par rapport à la phase solide.

3. Procédé selon la revendication 1 ou 2, dans lequel, au niveau de l'étape (a), un tampon de liaison est ajouté à l'échantillon contenant de l'ADN afin de préparer le mélange de liaison, dans lequel le tampon de liaison comprend l'au moins un polymère de poly(oxyde d'alkylène) qui est un polyéthylène glycol, l'au moins un cation divalent et au moins un agent de tamponnage, dans lequel ledit tampon de liaison présente un pH qui se situe à l'intérieur de la plage qui va de 8,5 à 9,5, et dans lequel, en option, le tampon de liaison comprend de façon additionnelle l'au moins un sel de métaux alcalins ou de métal alcalin.

4. Procédé selon la revendication 2 ou 3, dans lequel, au niveau de l'étape (c), un tampon de liaison est ajouté au reste de l'échantillon afin de préparer le second mélange de liaison, dans lequel le tampon de liaison comprend l'au moins un polymère de poly(oxyde d'alkylène) qui est un polyéthylène glycol, au moins un cation divalent et au moins un agent de tamponnage et dans lequel ledit tampon de liaison présente un pH qui se situe à l'intérieur de la plage qui va de 8,5 à 9,5, et dans lequel, en option, le tampon de liaison comprend de façon additionnelle au moins un sel de métaux alcalins ou de métal alcalin.

5. Procédé selon la revendication 4, dans lequel le même tampon de liaison est utilisé tel qu'au niveau de l'étape (a).

6. Procédé selon l'une quelconque des revendications 1 à 5, dans lequel le sel de métaux alcalins ou de métal alcalin présente une ou plusieurs des caractéristiques qui suivent :
a) le sel de métaux alcalins ou de métal alcalin est un halogénure de métaux alcalins ou de métal alcalin ;
b) le sel de métaux alcalins ou de métal alcalin est le chlorure de sodium ; et/ou
c) le sel de métaux alcalins ou de métal alcalin est présent dans le mélange de liaison selon une concentration qui est sélectionnée parmi les plages qui vont de 250 mM à 2 M, de 350 mM à 1,75 M, de 500 mM à 1,5 M, de 600 mM à 1,3 M et de 650 mM à 1,25 M.

7. Procédé selon l'une quelconque des revendications 1 à 6, dans lequel le cation divalent présente une ou plusieurs des caractéristiques qui suivent :
a) le cation divalent est sélectionné parmi Mg²⁺, Fe²⁺, Ca²⁺, Mn2²⁺, Zn²⁺ et Ni²⁺ et de préférence, il s'agit de Mg²⁺ ;
b) le cation divalent est présent dans le mélange de liaison sous la forme d'un sel dissous, dans lequel ledit sel est en option un halogénure ;
c) le cation divalent est présent dans le mélange de liaison sous la forme d'un sel dissous qui est le chlorure de magnésium ;
d) le cation divalent est présent dans le mélange de liaison selon une concentration qui est sélectionnée parmi les plages qui vont de 4 mM à 50 mM, de 5 mM à 40 mM, de 5,5 mM à 35 mM, de 6 mM à 30 mM, de 6,5 mM à 25 mM, de 7 mM à 20 mM et de 7,5 mM à 15 mM ; et/ou
e) le cation divalent est présent dans le mélange de liaison sous la forme d'un sel dissous et le mélange de liaison contient de façon additionnelle du chlorure de sodium.

8. Procédé selon l'une quelconque des revendications 1 à 7, présentant une ou plusieurs des caractéristiques qui suivent :
a) le polyéthylène glycol présente un poids moléculaire qui est sélectionné parmi les plages qui vont de 2 000 à 40 000, de 2 500 à 30 000, de 3 000 à 25 000, de 3 500 à 20 000, de 4 000 à 16 000, de 4 500 à 12 000 et de 5 000 à 10 000 ;
b) la valeur de coupure est réglée au moyen de la concentration du polyéthylène glycol dans le mélange de liaison ; et/ou
c) le mélange de liaison comprend le polyéthylène glycol selon une concentration qui s'inscrit à l'intérieur de la plage qui est sélectionnée parmi de 6 % à 20 %, de 7 % à 17 %, de 7,5 % à 15 %, de 8 % à 13 % et de 8,5 % à 12,5 %.

9. Procédé selon l'une quelconque des revendications 1 à 8, dans lequel le mélange de liaison présente une valeur de pH qui se situe à l'intérieur de la plage qui est sélectionnée parmi de 8,6 à 9,2 et/ou dans lequel le mélange liaison comprend au moins un agent de tamponnage, de préférence selon une concentration qui est sélectionnée parmi de 25 mM à 600 mM, de 50 mM à 500 mM, de 60 mM à 450 mM, de 70 mM à 400 mM, de 80 mM à 350 mM, de 90 mM à 300 mM et de 100 mM à 275 mM.

10. Procédé selon l'une quelconque des revendications 3 à 9, dans lequel les conditions de liaison sont établies de façon exclusive au moyen du tampon de liaison qui est mis en contact avec l'échantillon contenant de l'ADN et/ou avec le reste de l'échantillon.

11. Procédé selon l'une quelconque des revendications 3 à 5 ou procédé selon l'une quelconque des revendications 6 à 10 lorsqu'elle dépend de l'une quelconque des revendications 3 à 5, dans lequel le tampon de liaison présente une ou plusieurs des caractéristiques qui suivent :
a) il présente une valeur de pH qui se situe à l'intérieur de la plage qui est sélectionnée parmi de 8,6 à 9,2 ;
b) il comprend un agent de tamponnage selon une concentration qui est sélectionnée parmi de 25 mM à 1 M, de 50 mM à 750 mM, de 75 mM à 700 mM, de 100 mM à 650 mM, de 125 mM à 600 mM, de 150 mM à 550 mM, de 175 mM à 525 mM et de 200 mM à 500 mM ;
c) il comprend le sel de métaux alcalins ou de métal alcalin selon une concentration qui est sélectionnée parmi de 0,5 M à 4 M, de 0,7 M à 3,5 M, de 1 M à 3 M, de 1,2 M à 2,75 M et de 1,3 M à 2,5 M ; et
d) il comprend le polyéthylène glycol selon une concentration qui est sélectionnée parmi de 7 % à 45 %, de 10 % à 40 %, de 12,5 % à 35 %, de 15 % à 30 %, de 17,5 % à 27,5 % et de 20 % à 25 %.

12. Procédé selon l'une quelconque des revendications 3 à 5 ou procédé selon l'une quelconque des revendications 6 à 10 lorsqu'elle dépend de l'une quelconque des revendications 3 à 5, dans lequel le tampon de liaison présente les caractéristiques qui suivent :
i) il comprend le Mg²⁺ en tant que cation divalent, de préférence en tant que MgCl₂ ;
ii) il comprend du polyéthylène glycol selon une concentration qui est sélectionnée parmi de 12,5 % à 35 %, de 15 % à 30 %, de 17,5 % à 27,5 % et de 20 % à 25 %;
iii) il comprend le sel de métaux alcalins ou de métal alcalin, de préférence le chlorure de sodium, selon une concentration qui se situe à l'intérieur de la plage qui va de 0,5 M à 2,75 M ;
iv) il comprend l'au moins un agent de tamponnage selon une concentration qui se situe à l'intérieur de la plage qui va de 100 mM à 550 mM ; et
v) il présente un pH qui se situe à l'intérieur de la plage qui va de 8,5 à 9,5 ou de 8,6 à 9,2.

13. Procédé selon l'une quelconque des revendications 1 et 3 à 12, dans lequel le procédé est réalisé comme suit :
(a) préparation d'un mélange de liaison qui comprend l'échantillon contenant de l'ADN, un polyéthylène glycol selon une concentration qui se situe à l'intérieur de la plage qui va de 7,5 % à 15 %, de préférence de 8 % à 13 %, et du MgCl₂ selon une concentration qui se situe à l'intérieur de la plage qui va de 7 mM à 40 mM, de préférence de 8 mM à 20 mM, dans lequel ledit mélange de liaison présente un pH qui se situe à l'intérieur de la plage qui va de 8,5 à 9,25, et la liaison de molécules d'ADN précipitées selon une phase solide qui comporte une surface contenant du silicium non modifiée, d'où ainsi l'obtention d'une phase solide à laquelle sont liées les molécules d'ADN qui présentent une taille au-delà de la valeur de coupure, dans lequel, sous les conditions de liaison utilisées, les molécules d'ADN qui présentent une taille qui est inférieure à la valeur de coupure ne sont pas liées de façon prédominante selon la phase solide ; et
(b) la séparation des molécules d'ADN liées vis-à-vis du reste de l'échantillon ;
- en option, le lavage des molécules d'ADN liées ; et
- en option, l'élution des molécules d'ADN liées par rapport à la phase solide.

14. Procédé selon l'une quelconque des revendications 2 à 13, dans lequel le procédé est réalisé comme suit :
(a) préparation d'un premier mélange de liaison qui comprend l'échantillon contenant de l'ADN, du polyéthylène glycol selon une concentration qui se situe à l'intérieur de la plage qui va de 8,5 % à 9,5 % et du MgCl₂ selon une concentration qui se situe à l'intérieur de la plage qui va de 7 mM à 40 mM, de préférence de 8 mM à 20 mM, dans lequel ledit premier mélange de liaison présente un pH qui se situe à l'intérieur de la plage qui va de 8,5 à 9,25, et la liaison de molécules d'ADN précipitées selon une phase solide qui comporte une surface contenant du silicium non modifiée, d'où ainsi l'obtention d'une phase solide à laquelle sont liées les molécules d'ADN qui présentent une taille au-delà de la valeur de coupure supérieure, dans lequel, sous les conditions de liaison utilisées, les molécules d'ADN cibles qui présentent une taille qui est inférieure à la valeur de coupure supérieure ne sont pas liées de façon prédominante selon la phase solide ;
(b) la séparation des molécules d'ADN liées vis-à-vis du reste de l'échantillon qui comprend les molécules d'ADN cibles ;
(c) la préparation d'un second mélange de liaison qui comprend le reste de l'échantillon, dans lequel ledit second mélange de liaison présente un pH qui se situe à l'intérieur de la plage qui va de 8,5 à 9,25 et dans lequel la concentration du polyéthylène glycol dans le second mélange de liaison est augmentée par comparaison avec le premier mélange de liaison et se situe à l'intérieur de la plage qui va de 10 % à 12 %, et la liaison de molécules d'ADN cible cibles précipitées selon une phase solide qui comporte une surface contenant du silicium non modifiée, d'où ainsi l'obtention d'une phase solide à laquelle sont liées les molécules d'ADN cibles, dans lequel, sous les conditions de liaison utilisées, les molécules d'ADN qui présentent une taille qui est inférieure à la valeur de coupure inférieure ne sont pas liées de façon prédominante selon la phase solide ; et
(d) la séparation des molécules d'ADN cibles liées vis-à-vis du reste de l'échantillon;
- en option, le lavage des molécules d'ADN cibles liées ; et
- en option, l'élution des molécules d'ADN cibles liées par rapport à la phase solide.

15. Procédé selon l'une quelconque des revendications 1 à 14, pour isoler des molécules d'ADN ligaturées à un/des adaptateur(s) en tant que molécules d'ADN cibles vis-à-vis d'un échantillon contenant de l'ADN qui est un échantillon de ligation d'adaptateur(s) et pour enlever des monomères d'adaptateur(s) et des produits de ligation adaptateur-adaptateur, dans lequel les molécules d'ADN ligaturées à un/des adaptateur(s) sont séparées de monomères d'adaptateur(s) non ligaturés et de produits de ligation adaptateur-adaptateur sur la base de la taille plus importante des molécules d'ADN ligaturées à un/des adaptateur(s).

16. Procédé selon l'une quelconque des revendications 1 à 15, dans lequel la phase solide présente une ou plusieurs des caractéristiques qui suivent :
a) la phase solide fournit une surface siliceuse ;
b) la phase solide fournit une surface en silice ;
c) la phase solide est fournie sous la forme d'une membrane, d'un filtre, d'une feuille ou de particules ; et
d) la phase solide est une colonne qui est basée sur des particules magnétiques ou qui est fournie sous la forme de particules magnétiques.

17. Kit pour l'isolement sélectif en termes de taille de molécules d'ADN cibles qui présentent une taille au-delà d'une valeur de coupure souhaitée vis-à-vis d'un échantillon contenant de l'ADN, dans lequel le kit est approprié pour le procédé selon la revendication 1, le kit comprenant :
(a) un tampon de liaison qui comprend au moins un polymère de poly(oxyde d'alkylène) qui est un polyéthylène glycol, au moins un sel de métaux alcalins ou de métal alcalin, au moins un cation divalent et au moins un agent de tamponnage, dans lequel le tampon de liaison présente une valeur de pH qui se situe à l'intérieur de la plage qui va de 8,5 à 9,5 ;
(b) une phase solide qui comporte une surface contenant du silicium non modifiée ;
(c) en option, au moins une solution de lavage ; et
(d) en option, au moins une solution d'élution.

18. Kit selon la revendication 17, dans lequel le tampon de liaison présente une ou plusieurs des caractéristiques telles que spécifiées selon la revendication 11 et/ou dans lequel la phase solide présente une ou plusieurs des caractéristiques qui ont été définies selon la revendication 16.
